(19) Europäisches Patentamt
European Patent Office
Office européen des brevets

(11) EP 1 869 108 B1

(12) **EUROPÄISCHE PATENTSCHRIFT**

(45) Veröffentlichungstag und Bekanntmachung des Hinweises auf die Patenterteilung:
**18.09.2013 Patentblatt 2013/38**

(21) Anmeldenummer: **06753377.8**

(22) Anmeldetag: **30.03.2006**

(51) Int Cl.:
***D06M 15/643*** (2006.01) ***A61K 8/898*** (2006.01)
***A61Q 5/02*** (2006.01) ***A61Q 19/00*** (2006.01)
***C08G 77/46*** (2006.01)

(86) Internationale Anmeldenummer:
**PCT/EP2006/002910**

(87) Internationale Veröffentlichungsnummer:
**WO 2006/103075 (05.10.2006 Gazette 2006/40)**

(54) **POLYAMINO- UND/ODER POLYAMMONIUM-POLYSILOXAN-COPOLYMER-VERBINDUNGEN MIT KAMMARTIG ANGEORDNETEN POLYALKYLENOXIDEINHEITEN**

POLYAMINO AND/OR POLYAMMONIUM/POLYSILOXANE COPOLYMER COMPOUNDS WITH POLYALKYLENE OXIDE UNITS IN COMB-SHAPED ARRANGEMENT

COMPOSÉS COPOLYMÈRES DE POLYAMINO ET / OU POLYAMMONIUM / POLYSILOXANE À UNITÉS OXYDE DE POLYALKYLÈNE DISPOSÉES EN FORME DE PEIGNE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **30.03.2005 DE 102005014311**

(43) Veröffentlichungstag der Anmeldung:
**26.12.2007 Patentblatt 2007/52**

(73) Patentinhaber: **Momentive Performance Materials GmbH**
**51368 Leverkusen (DE)**

(72) Erfinder:
- **MOELLER, Annette**
  **51381 Leverkusen (DE)**
- **ROOS, Christopher**
  **51065 Köln (DE)**
- **WAGNER, Roland**
  **53721 Siegburg (DE)**
- **SOCKEL, Karl-Heinz**
  **51373 Leverkusen (DE)**
- **STACHULLA, Karl-Heinz**
  **51379 Leverkusen (DE)**
- **WITOSSEK, Anita**
  **40764 Langenfeld (DE)**
- **LANGE, Horst**
  **44879 Boghum (DE)**

(74) Vertreter: **Gille Hrabal**
**Postfach 18 04 09**
**40571 Düsseldorf (DE)**

(56) Entgegenhaltungen:
**WO-A-2006/067225 DE-A1- 10 214 290**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 869 108 B1

## Beschreibung

Die Erfindung betrifft Polyamino- Polysiloxan- und/ oder Polyammonium- Polysiloxan- Copolymer- Verbindungen mit kammartig angeordneten Polyalkylenoxideinheiten, Verfahren zu ihrer Herstellung und ihre Verwendung, insbesondere zur Behandlung von Textilien und anderen natürlichen und synthetischen faserartigen Materialien.

Aminogruppen enthaltende Polysiloxane sind als textile Weichmacher bekannt (EP 441 530).

Es ist weiterhin bekannt, α, ω- epoxymodifizierte Siloxane mit α, ω- aminofunktionalisierten Alkylenoxiden umzusetzen, und diese Produkte als hydrophile Weichmacher einzusetzen (US 5, 807, 956, US 5, 981, 681) . In Weiterentwicklung diesen Gedankens sind Blockcopolymere vorgeschlagen worden, in denen α, ω- epoxymodifizierten Siloxane und α, ω- epoxymodifizierte Polyether über primäre Alkylamine als Kettenverlängerer miteinander verbunden werden. (US 6, 475, 568) . Als Vorteile dieser Verbindungen werden eine höhere Weichheit und Substratbenetzungsfähigkeit genannt.

Aminosiloxane mit Ethylenoxid- / Propylenoxideinheiten in den Seitenketten sind ebenfalls beschrieben worden (US 5, 591, 880, US 5, 650, 529) .

Eine gesteigerte Substantivität wird von Polysiloxanquats ("Polysiloxanquats" = quaternäre Ammoniumgruppen-enthaltende Polysiloxane) erwartet. Die Reaktion von α,ω-Diepoxiden mit tertiären Aminen in Gegenwart von Säuren liefert α,ω-diquartäre Siloxane, welche zu Haarpflegezwecken eingesetzt werden können (DE-A-37 19 086). Neben tetraalkylsubstituierten quartären Ammoniumstrukturen werden auch aromatische Imidazoliniumderivate beansprucht.

Eine Verringerung der Auswaschbarkeit aus Haaren kann erzielt werden, wenn die α, ω- Diepoxide mit ditertiären Aminen in Gegenwart von Säuren zu langkettigen polyquartären Polysiloxanen umgesetzt werden (EP- A-282720) . Aromatische quartäre Ammoniumstrukturen werden nicht offenbart.

Derartige polyquaternäre Imidazoliniumderivate werden in US 6,240,929 behandelt. Diese kationischen Verbindungen sollen eine weiter erhöhte Kompatibilität gegenüber den in kosmetischen Formulierungen vorhandenen anionischen Tensiden besitzen. Allerdings bezieht sich die Auswaschbeständigkeit aus Haaren auf den kurzzeitigen Angriff von vornehmlich Wasser und sehr milden, die Haut nicht irritierenden Tensiden, während waschbeständige, hydrophile Weichmacher für Textilien dem Angriff konzentrierter Tensidlösungen mit hohem Fett- und Schmutzlösevermögen zu widerstehen haben. Erschwerend kommt hinzu, daß moderne Waschmittel stark alkalische Komplexbildner, oxydativ wirkende Bleichmittel und komplexe Enzymsysteme enthalten und die Fasern der Einwirkung oftmals über Stunden bei erhöhten Temperaturen ausgesetzt sind.

Durch Einführung von Alkylenoxidgruppen zusätzlich zu den Quatstrukturen soll die Hydrophilie gesteigert werden.

Streng kammartige alkylenoxidmodifizierte Polysiloxanquats sind ebenfalls beschrieben worden. Die Hydroxylgruppen von kammartig substituierten Polyethersiloxanen werden mit Epichlorhydrin (US 5,098,979) oder Chloressigsäure (US 5,153,294, US 5,166,297) in die entsprechenden Chlorderivate überführt. Anschließend erfolgt eine Quaternierung mit tertiären Aminen. Nachteil der Lösungen gemäß US 5,098,979, US 5,153,294 und US 5,166,297 ist, daß die Quatgruppen durch die Alkylenoxideinheiten weit von der Hauptkette beabstandet positioniert sind, wodurch die Substantivität des Gesamtmoleküls geschwächt wird.

Verzweigte alkylenoxidmodifizierte Polysiloxanquats sind aus α, ω- OH- terminierten Polysiloxanen und Trialkoxysilanen durch Kondensation synthetisiert worden. Die quartäre Ammoniumstruktur wird über das Silan eingebracht, wobei das quartäre Stickstoffatom durch Alkylenoxideinheiten substituiert ist (US 5, 602, 224) . Nachteil dieser Lösung ist die wenig flexible Kopplung von Quatgehalt und Verzweigungsgrad.

In US 6,242,554 werden α,ω-difunktionelle Siloxanderivate beschrieben, die jeweils über eine separate quartäre Ammonium- und Alkylenoxideinheit verfügen. Diese monoquaternären Verbindungen sich allerdings nicht hinreichend substantiv.

In den WO 02/10257 und WO 02/10259 werden polyquaternäre Polysiloxanblockcopolymere als fortschrittliche Weichmacher beansprucht, die ein Weichmachen von Textilien während der Ausrüstung und auch alternativ während des Waschprozesses aus Detergenzienformulierungen heraus ermöglichen. Die US-A 2002/0103094 behandelt die Verwendung der genannten Siliconmaterialien in Textilpflegeformulierungen. Die WO 02/10257, WO 02/10259 und US-A 2002/0103094 offenbaren Materialien, in denen die Quatgruppen anteilig oder vollständig separiert von den Alkylenoxideinheiten vorliegen. Diese Alkylenoxideinheiten werden als α,ω-difunktionelle Einheiten in das Blockcopolymer eingebaut.

In WO 03/78504 werden verzweigte polyquaternäre Polysiloxanblockcopolymere als permanente Textilweichmacher beschrieben. Als verzweigende Einheit können u.a. trifunktionelle Alkylenoxidstrukturen eingebaut werden, die von den Quatgruppen separiert vorliegen. Bedingt durch ihren Vernetztercharakter können diese trifunktionellen Alkylenoxide nur in begrenztem Umfang eingeführt werden.

DE 102 14 290 beschreibt Polyammonium-Polysiloxan-Copolymere mit Polyalkylenoxidblöcken in der Polymerhauptkette.

Ausgehend von diesem Stand der Technik ist eine weitere Verbesserung der Hydrophilie der siliconbasierten

Blockcopolymeren ohne Preisgabe der erreichbaren Weichheit der behandelten Fasern, insbesondere bei gleichbleibender oder verbesserter Substantivität (Haftung der Siloxansysteme auf der Faser), der Flexibilität bei der Formulierung der Siloxansysteme und der Darreichungsform insbesondere in Richtung auf eine Verringerung der notwendigen Einsatzmengen und der Materialkosten sehr wünschenswert.

Es ist somit eine Aufgabe der Erfindung, polyquaternierte Siloxanblockcopolymere zur Behandlung von Textilien und anderen natürlichen und synthetischen faserartigen Materialien wie z.B. Papierfasern, Wolle und Haare bereitzustellen, die derartigen Materialien bzw. Substraten, bevorzugt Textilmaterialien, eine silicontypische Weichheit, eine verbesserte Elastizität und verringerte Knitterneigung bei gesteigerter Hydrophilie verleihen.

Es ist eine weitere Aufgabe der Erfindung, die Verwendung der erfindungsgemäßen Substanzen als Bestandteil von Systemen zur textilen Erstausrüstung, als Weichmacher in auf anionischen und/oder nichtionischen Tensiden beruhenden Waschmittelsystemen, in separaten Weichmachersystemen zur Faserbehandlung nach der Durchführung der Faserwäsche, als Bestandteil von Weichmachersystemen für Vliese, wie Papier und Textilien, als Bügelhilfe und Mittel zur Verhinderung bzw. Rückgängigmachung von Textilverknitterungen, als Bestandteil von Formulierungen zur Behandlung harter Oberflächen, wie Glas Keramik, Kunststoff, beispielsweise Automobilen, sowie als Bestandteil von kosmetischen Systemen zur Behandlung von Haaren und Haut zu beschreiben.

Es wurde überraschend gefunden, daß Polyamino- Polysiloxan- und/ oder Polyammonium- Polysiloxan- Copolymer- Verbindungen, dadurch gekennzeichnet, dass sie Wiederholungseinheiten der Formel (I) aufweisen:

$$- [Q- V]- \qquad (I)$$

worin Q aus der Gruppe ausgewählt wird, die besteht aus:

$-NR-$,
$-N^{+}R_{2}-$

einem gesättigten oder ungesättigten diaminofunktionellen Heterocyclus der Formeln:

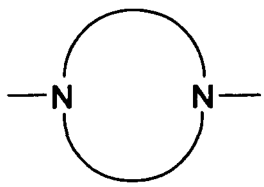
,

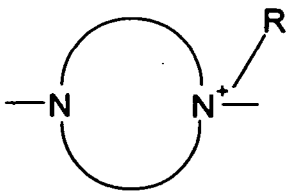

und

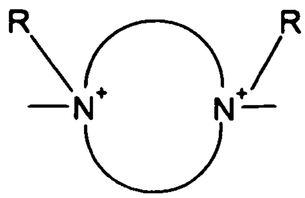
,

sowie
einem aromatischen diaminofunktionellen Heterocyclus der Formel:

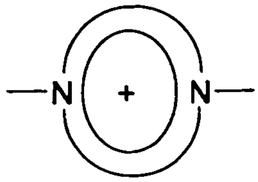

einem dreiwertigen Rest der Formel:

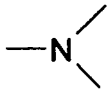

,

einem dreiwertigen Rest der Formel:

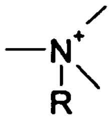

,

oder
einem vierwertigen Rest der Formel,

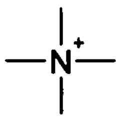

worin R jeweils Wasserstoff oder einen einwertigen organischen Rest darstellt,
wobei Q nicht an ein Carbonylkohlenstoffatom bindet,
V aus der Gruppe ausgewählt wird, die aus $V^1$, $V^2$ und $V^3$ besteht, worin
$V^2$ ausgewählt wird aus zweiwertigen, geradkettigen, cyclischen oder verzweigten, gesättigten, ungesättigten oder aromatischen Kohlenwasserstoffresten mit bis zu 1000 Kohlenstoffatomen (wobei die Kohlenstoffatome des unten definierten Polysiloxanrestes $Z^2$ nicht mitgezählt werden) , die gegebenenfalls eine oder mehrere Gruppen, ausgewählt aus
- O-, -CONH-,

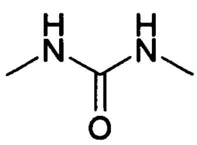

,

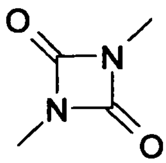

,

- $CONR^2$-, worin $R^2$ Wasserstoff, einen einwertigen, geradkettigen, cyclischen oder verzweigten, gesättigten, ungesättigten oder aromatischen Kohlenwasserstoffrest mit bis zu 100 Kohlenstoffatomen darstellt, der eine oder mehrere Gruppen ausgewählt aus- O-, -NH-, -C (O)- und- C (S)- enthalten kann, und der gegebenenfalls durch eine oder mehrere Substituenten, ausgewählt aus der Gruppe, die besteht aus einer Hydroxylgruppe, einer gegebenenfalls substituierten, bevorzugt ein oder mehrere Stickstoffatome enthaltenden heterocyclischen Gruppe, Amino, Alkylamino, Dialkylamino, Ammonium, Polyetherresten und Polyetheresterresten substituiert sein kann, wobei wenn mehrere Gruppen- $CONR^2$ vorliegen, diese gleich oder verschieden sein können,
- C (O)- und- C (S)- enthalten kann,
der Rest $V^2$ gegebenenfalls durch eine oder mehrere Hydroxylgruppen und/ oder durch
- Si $(OR)_{3-a}$ $(R')_a$
worin a eine ganze Zahl von 0 bis 2 ist und R und R' gleich oder verschieden voneinander sein können und jeweils einen organischen Rest darstellen, substituiert sein kann, und
der Rest $V^2$ mindestens eine Gruppe- $Z^2$- der Formel

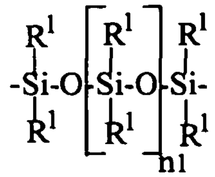

enthält, worin $R^1$ gleich oder verschieden sein kann und aus der Gruppe ausgewählt wird, die besteht aus: $C_1$ bis $C_{22}$ Alkyl, Fluor ($C_1$-$C_{10}$) alkyl, $C_6$- $C_{10}$ Aryl und- W- Si $(OR)_{3-a}$ $(R')_a$ besteht, worin R, R' und a wie oben definiert sind und W- O- oder einen zweiwertigen, geradkettigen, cyclischen oder verzweigten, gesättigten, ungesättigten oder aromatischen Kohlenwasserstoffrest mit bis zu 100 Kohlenstoffatomen darstellt, der eine oder mehrere Gruppen- C (O)-, -O-, NH-, -S- enthalten kann, und gegebenenfalls durch Hydroxygruppen substituiert sein kann, und
$n_1$ = 20 bis 1000 bedeutet,
V1 ausgewählt wird aus zweiwertigen, geradkettigen, cyclischen oder verzweigten, gesättigten, ungesättigten oder aromatischen Kohlenwasserstoffresten mit bis zu 1000 Kohlenstoffatomen, die gegebenenfalls eine oder mehrere Gruppen, ausgewählt aus
- O-, -CONH-,

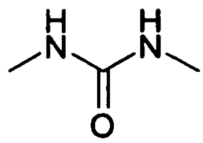 , 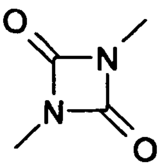 ,

- CONR$^2$-, worin $R^2$ wie oben definiert ist, wobei die Gruppen $R^2$ in den Gruppen V $^1$ und $V^2$ gleich oder verschieden sein können,
- C (O)-, -C (S)- und- $Z^1$- enthalten kann, worin- $Z^1$- eine Gruppe der Formel

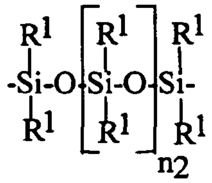

ist, worin
$R^1$ wie oben definiert ist, wobei die Gruppen $R^1$ in den Gruppen $V^1$ und $V^2$ gleich oder verschieden sein können, und
$n_2$ = 0 bis 19 bedeutet,
und der Rest $V^1$ gegebenenfalls durch eine oder mehrere Hydroxylgruppen und/ oder durch

- Si $(OR)_{3-a}$ $(R')_a$

worin a eine ganze Zahl von 0 bis 2 ist und R und R' gleich oder verschieden voneinander sein können und jeweils einen organischen Rest darstellen, substituiert sein kann, und
$V^3$ einen drei- oder höherwertigen, geradkettigen, cyclischen oder verzweigten, gesättigten, ungesättigten oder aromatischen Kohlenwasserstoffrest mit bis zu 1000 Kohlenstoffatomen darstellt, der gegebenenfalls eine oder mehrere Gruppen, ausgewählt aus
- O-, -CONH-,

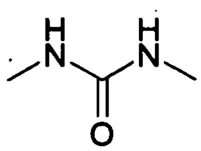 , 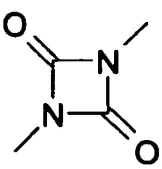 ,

- CONR$^2$-, worin $R^2$ wie oben definiert ist, -C (O)-, -C (S)-, -$Z^1$-, das wie oben definiert ist, -$Z^2$- das wie oben definiert ist, und $Z^3$, worin $Z^3$ eine drei- oder höherwertige Organopolysiloxaneinheit ist, enthalten kann, und
der gegebenenfalls durch eine oder mehrere Hydroxylgruppen und/ oder durch
- Si $(OR)_{3-a}$ $(R')_a$
worin a eine ganze Zahl von 0 bis 2 ist und R und R' gleich oder verschieden voneinander sein können und jeweils einen organischen Rest darstellen, substituiert sein kann, substituiert sein kann,
mit der Maßgabe,

- dass die genannte Polysiloxan-Verbindung mindestens eine Gruppe -$Z^1$-, -$Z^2$- oder $Z^3$ enthalten,
- dass die drei- und vierwertigen Reste Q entweder der Verzweigung der aus Q und V gebildeten Hauptkette dienen, so dass die Valenzen, die nicht der Bindung in der Hauptkette dienen, weitere aus- [Q- V]- Einheiten gebildete Verzweigungen tragen, oder die drei- und vierwertigen Reste Q sättigen sich mit Resten $V^3$ innerhalb einer linearen Hauptkette ohne Bildung einer Verzweigung ab, und
- dass Gruppen Q enthalten sind, die mindestens ein Resten R aufweisen, der einen polyalkylenoxid-haltigen Rest $R^O$ darstellt,

und worin die aus Ammoniumgruppen resultierenden positiven Ladungen durch organische oder anorganische Säureanionen neutralisiert sind,
die vorstehend beschriebenen Aufgabenstellungen lösen können.

In einer bevorzugten Ausführungsform sind die erfindungsgemäßen Polyamino- Polysiloxan- und/ oder Polyammonium- Polysiloxan- Copolymer- Verbindungen dadurch gekennzeichnet, dass das molare Verhältnis $R^O$ : Q von 0, 001 bis 2, bevorzugter von 0.01 bis 1, bevorzugter 0, 05 bis 0, 8 beträgt. Wenn das molare Verhältnis weniger als 0, 001 beträgt ist die Hydrophilie zu gering. Eine optimale Abstimmung von Weichheit und Hydrophilie gelingt im Bereich von 0, 05 bis 0, 8.

Der Rest $R^O$ stellt bevorzugt eine Gruppe der Formel (III) dar:

- X- E- Y (III) ,

worin X eine Einfachbindung oder einen zweiwertigen, geradkettigen, verzweigten oder cyclischen Kohlenwasserstoffrest mit bis zu 20 Kohlenstoffatomen darstellt, der gegebenenfalls Stickstoff und/ oder Sauerstoff enthalten kann, und X über ein Kohlenstoffatom mit dem Stickstoffatom von Q verbunden ist,
E einen Polyalkylenoxidrest der Formel

$$-[(C_aH_{2a})O]_y-$$

worin a = 2 bis 4 ist, und
y = 2 bis 10000 ist,
der über ein Kohlenstoffatom mit der Gruppe X und über ein Sauerstoffatom mit der Gruppe Y verbunden ist,
Y Wasserstoff oder einen einwertigen, geradkettigen, verzweigten oder cyclischen, gesättigten, ungesättigen oder aromatischen Kohlenwasserstoffrest mit bis zu 24 Kohlenstoffatomen darstellt, der Sauerstoff und/ oder Stickstoff und/ oder Halogen enthalten kann und über ein Kohlenstoffatom mit der Gruppe E verbunden ist.

Bevorzugt ist $R^O$ eine Gruppe der Formel (III), in der -E- eine Gruppe der Formel (IV) darstellt:

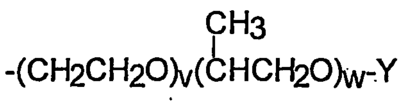

(IV),

wobei es sich um statstische und blockartige Sequenzen der Ethylen- und Propylenoxid-Einheiten handeln kann und die Bindung an E über eine Ethylen- oder Propylenoxid-Einheit erfolgen kann, die Darstellung der Gruppe (IV) mithin nur quantitativ ist,
mit
v = 0 bis 200,
w = 0 bis 200,
v+w≥1.

In der Gruppe der Formel (III) wird weiterhin Y bevorzugt aus H oder geradkettigen, cyclischen, verzweigten $C_1$ bis $C_{22}$ Alkyl-, Alkenyl-, Alkinyl-, Fluor ($C_1$- $C_{10}$) alkyl- und $C_6$- $C_{10}$ Arylresten ausgewählt.

Weitere bevorzugte Alkylenoxideinheiten $R^O$ weisen bevorzugt die Struktur auf:

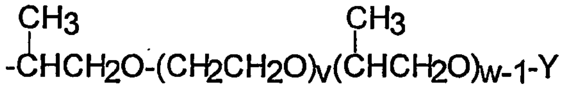

mit
v = 0 bis 200,
w = 0 bis 200,
v+w≥1,
Y = H oder geradkettiger, cyclischer, verzweigter $C_1$- bis $C_{22}$- Alkyl-, Alkenyl, Alkinyl-, Fluor- ($C_1$- $C_{10}$)- alkyl- und $C_6$- $C_{10}$- Arylrest.

Bevorzugt sind in den obigen allgemeinen Polyalkylenoxidformeln:

v 0 bis 100, besonders bevorzugt 0 bis 70, speziell 0 bis 40, ganz speziell 0 bis 20,
w 0 bis 100, besonders bevorzugt 0 bis 70, speziell 0 bis 40, ganz speziell 0 bis 20,
Y ein geradkettiger, cyclischer, verzweigter $C_1$ bis $C_{12}$- Alkyl-, Alkenyl, Alkinyl-, oder $C_6$- $C_{10}$- Arylrest, speziell Methyl, Ethyl, Isopropyl, Butyl, Hexyl, Dodecyl, Allyl, Oleyl, Phenyl.

Eine weitere bevorzugte Alkylenoxideinheit $R^O$ weist die Struktur

- ($C_1$- $C_{12}$)- Alkylen- $N^+R_2EY$

auf, worin $C_1$- $C_{12}$- Alkylen eine geradkettige, cyclische oder verzweigte Alkyleneinheit mit 1 bis 12 Kohlenstoffatomen ist, und R, E und Y wie oben definiert sind.

Die Polysiloxan-Verbindungen, die im Mittel mindestens zwei, bevorzugt mindestens drei, noch bevorzugter mindestens vier Einheiten der Formel (I) enthalten, wobei bevorzugt im Mittel mindestens zwei, bevorzugter mindestens drei, noch bevorzugter mindestens vier Einheiten $R^O$, und im Mittel mindestens eine Einheit $V^1$, $V^2$ und/oder $V^3$ enthalten sind, werden bevorzugt durch monofunktionelle Gruppen -Q-R und/oder -V-R, d.h. z.B. durch Aminogruppen terminiert. Diese ergeben sich durch Absättigung einer der beiden Bindungsstellen von Q oder V durch eine einwertige Gruppe R oder Wasserstoff, die wie oben definiert ist, und werden nachfolgend auch $V^{st}$ oder $Q^{st}$ genannt. An Stelle von $V^{st}$ können auch andere, nicht umgesetzte Reaktivgruppen, wie Epoxy- oder Halogenalkylgruppen stehen.

Bei den erfindungsgemäßen Polysiloxan- Verbindungen, die im Mittel mindestens zwei Einheiten der Formel (I) enthalten, wobei im Mittel mindestens zwei Einheiten $R^O$ und im Mittel mindestens eine Einheit $V^1$, $V^2$ und/ oder $V^3$ enthalten sind, handelt es sich zum Beispiel um lineare Polysiloxancopolymere der allgemeinen Formel (I') :

- [Q- V]- (I')

worin Q wie oben definiert ist,
V und mindestens eine Gruppe $V^1$ oder Gruppe $V^2$ darstellt, worin $V^1$ und $V^2$ wie oben definiert sind. Zusätzlich kann V auch drei- oder höherwertige, besonders dreiwertige Reste $V^3$ darstellen. In diesem Fall liegen bevorzugt auch drei- oder vierwertige Einheiten Q, wie oben definiert vor, und die Absättigung der drei- oder höherwertigen Reste $V^3$ und der drei- oder vierwertige Einheiten Q erfolgt bevorzugt ausschließlich untereinander innerhalb der linearen Hauptkette unter Ausbildung cyclischer Strukturen, wie weiter unten eingehender erläutert. Dieser Fall ist jedoch weniger bevorzugt.

In den allgemeinen Formeln (I) bzw. (I') kann das molare Verhältnis der Gruppen $V^1$ und $V^2$ in den Polysiloxan-Verbindungen $V^2/V^1$ an sich einen beliebigen Wert annehmen. Erfindungsgemäß ist somit auch der Fall eingeschlossen, bei dem die Polysiloxanverbindung der Formeln (I) oder (I') nur $V^2$- Einheiten enthält, die Polysiloxanverbindung also die Formel- [Q- $V^2$]- aufweist. Auch der Fall, bei dem die Polysiloxanverbindung nur $V^1$- Einheiten. enthält, ist erfindungsgemäß umfasst. In diesem Fall müssen die $V^1$- Einheiten jedoch $Z^1$- Siloxaneinheiten enthalten.

In einer bevorzugten Ausführungsform der Erfindung enthält die Polysiloxanverbindung der Formeln (I) oder (I') jedoch sowohl $V^2$ als auch $V^1$-Einheiten.

In einer weiteren bevorzugten Ausführungsform der vorliegenden Erfindung beträgt das molare Verhältnis der Gruppen $V^1$ und $V^2$ in den Polysiloxan-Verbindungen der allgemeinen Formeln (I) bzw. (I'):

$$V^2/V^1 = 1.$$

In einer weiteren Ausführungsform der linearen Polysiloxanverbindungen der Formel (I) bzw. (I') ist $V^2/V^1$ ungleich 1, bevorzugt ist $V^2/V^1 < 1$, bevorzugter < 0,9, noch bevorzugter erfüllt $V^2/V^1$ die Beziehung

$$0{,}0005 < V^2/V^1 < 0{,}5,$$

noch bevorzugter

$$0{,}0005 < V^2/V^1 < 0{,}3.$$

Die Gruppe R wird bevorzugt ausgewählt aus den Gruppen $R^2$.

Bevorzugte Ausführungsformen von Q sind:

Für Reste der Formel

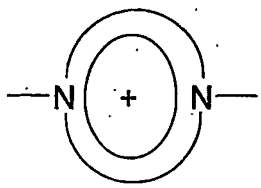

:

eine quaternierte Imidazoleinheit der Struktur

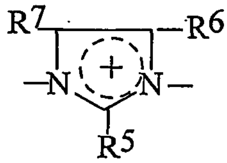

,

eine quaternierte Pyrazoleinheit der Struktur

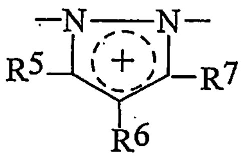

.

Für Reste der Formel

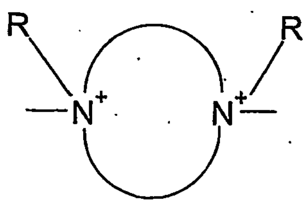

eine zweifach quaternierte Piperazineinheit der Struktur

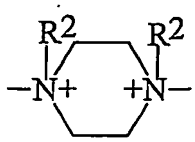

Für Reste der Formel

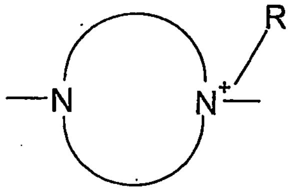

eine monoquaternierte Piperazineinheit der Struktur

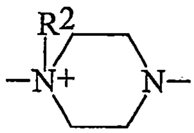

und
eine monoquaternierte Piperazineinheit der Struktur

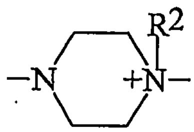

.

Für Reste der Formel

$- N^{+}R^{2}-$

eine zweifach quaternierte Einheit der Struktur

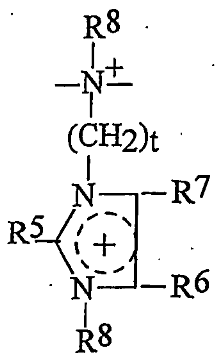

,

eine monoquaternierte Einheit der Struktur

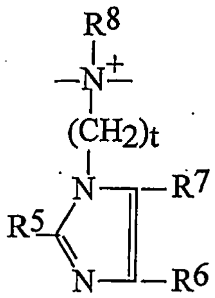

,

eine zweifach quaternierte Einheit der Struktur

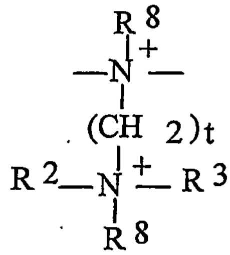

,

und eine monoquaternierte Einheit der Struktur

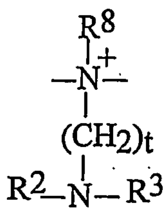

Für Reste der Formel

-NR-

eine monoquaternierte Einheit der Struktur

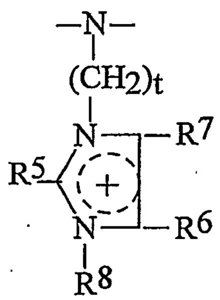

,

eine monoquaternierte Einheit der Struktur

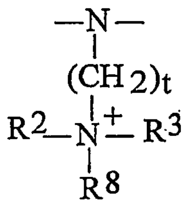

.

Worin:

t von 2 bis 10 ist,
R wie oben definiert, bevorzugt $R^2$ ist, $R^2$ wie oben definiert ist, und die Bedeutung von $R^2$ von der Bedeutung der obigen Gruppe $R^2$ gleich oder verschieden sein kann,
$R^3$ die Bedeutung von $R^2$ aufweist, wobei $R^2$ und $R^3$ gleich oder verschieden sein können, oder
$R^2$ und $R^3$ gemeinsam mit dem positiv geladenen Stickstoffatom einen fünf- bis siebengliedrigen Heterocyclus bilden, der gegebenenfalls zusätzlich ein oder mehrere Stickstoff-, Sauerstoff- und/oder Schwefelatome aufweisen kann,
$R^5$, $R^6$, $R^7$ gleich oder verschieden sein können und aus der Gruppe ausgewählt werden, die besteht aus: H, Halogen, Hydroxylgruppe, Nitrogruppe, Cyanogruppe, Thiolgruppe, Carboxylgruppe, Alkylgruppe, Monohydroxyalkylgruppe, Polyhydroxyalkylgruppe, Thioalkylgruppe, Cyanoalkylgruppe, Alkoxygruppe, Acylgruppe, Acetyloxygruppe, Cycloalkylgruppe, Arylgruppe, Alkylarylgruppe, und Gruppen des Typs $-NHR^W$, in denen $R^W$ H, Alkylgruppe, Monohydroxyalkylgruppe, Polyhydroxyalkylgruppe, Acetylgruppe, Ureidogruppe bedeuten, und jeweils zwei der benachbarten Reste $R^5$, $R^6$ und $R^7$ mit den sie an den Heterocyclus bindenden Kohlenstoffatomen aromatische Fünf- bis Siebenringe bilden können, und
$R^8$ die Bedeutung von $R^2$ aufweist, wobei $R^8$ und $R^2$ gleich oder verschieden sein können. Insbesondere kann R8 ein polyoxyalkylen-haltiger Rest sein, was zur Bildung eines $R^O$-haltigen Restes Q führt.

Im Falle, dass Q einen dreiwertigen Rest der Formeln

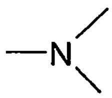

oder

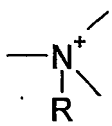

oder eine vierwertigen Rest

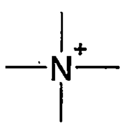

darstellt, dienen diese Reste bei den linearen Copolymeren der Formel (I') wie oben erwähnt bevorzugt nicht der Verzweigung der Polysiloxan-Copolymere sondern diese Reste sind ausschließlich mit insbesondere dreiwertigen Resten $V^3$ verbunden, wobei cyclische Strukturen ausgebildet werden, die Bestandteil der linearen Hauptkette sind, wie z.B. ein Strukturelement der Formel:

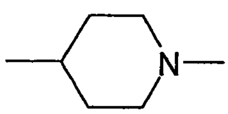

.

In einer bevorzugten Ausführungsform der Polysiloxanverbindungen der Formel (I) bzw. (I') stellt $V^2$ eine Gruppe der Formel

$$- V^{2*}- Z^2- V^{2*}-$$

dar, worin $Z^2$ wie oben definiert ist und $V^{2*}$ einen zweiwertigen geradkettigen, cyclischen oder verzweigten, gesättigten, ungesättigten oder aromatischen Kohlenwasserstoffrest mit bis zu 40 Kohlenstoffatomen darstellt, der gegebenenfalls eine oder mehrere Gruppen, ausgewählt aus- O-, -CONH-, -$CONR^2$-, worin $R^2$ wie oben definiert ist, -C (O)- und- C (S)- enthalten kann, und der Rest $V^{2*}$ gegebenenfalls durch eine oder mehrere Hydroxylgruppen substituiert sein kann.

In der vorstehend erwähnten Ausführungsform kann das erfindungsgemäße lineare Polysiloxancopolynier die folgenden Wiederholungseinheiten aufweisen:

- $[V^{2*}- Z^2- V^{2*}- Q]$- bevorzugt zusammen mit- $[V^1- Q]$- .

Das molare Verhältnis der Wiederholungseinheiten- $[V^{2*}- Z^2- V^{2*}- Q]$- zu- $[V^1- Q]$-, also das Verhältnis $V^2/V^1$ kann, wie vorstehend erwähnt, etwa 1 betragen, ist in einer Ausführungsform jedoch bevorzugt ungleich 1, bevorzugter > 1 noch bevorzugter > 1 und kleiner 1, 5. Die kammartige Einführung der hydrophilen Seitengruppen R° ermöglicht es, den Anteil der die weichmachenden Eigenschaften beisteuernden Gruppe $V^2$ bei gleichbleibender Hydrophilie zu erhöhen. Umgekehrt ist es bei gegebenem Anteil von weichmachenden Gruppen $V^2$ möglich, die Hydrophilie durch Einführung der Gruppe $R^O$ zu erhöhen.

Wie weiter unten im Zusammenhang mit dem Verfahren zur Herstellung der vorstehend beschriebenen linearen Polysiloxancopolymere noch ausführlich erläutert wird, können die blockartigen Sequenzen, die mehr als eine- $[V^1- Q]$- Einheit miteinander verknüpft aufweisen, je nach Herstellweise regelmäßig mit den $V^2$- Q- Einheiten oder unregelmäßig mit den $V^2$- Q- Einheiten verbunden werden.
Dies meint folgendes:

Bei der regelmäßigen Verbindung, bei der beispielsweise ein der Gruppe- Q- $[V^1- Q]_x$- entsprechendes Präpolymer mit $V^2$ entsprechenden Monomer- Einheiten im molaren Verhältnis 1: 1 umgesetzt wird, lassen sich die linearen Polysiloxancopolymere wie folgt darstellen:

$$- \{V^2- Q- [V^1- Q]_x- \}_y- .$$

x kann dabei 2 bis 2000 sein und ist der Mittelwert der Verteilung und y ist ebenfalls ein Mittelwert und beträgt 2 bis 1000.

Allgemein weisen daher die erfindungsgemäßen Polysiloxanpolymere bevorzugt die Formel

$$- [Q- V]_{y'}-,$$

worin y' 2 bis 1000, bevorzugter 3 bis 500, noch bevorzugter 4 bis 200 beträgt.

Die durch die Formel- $\{V^2- Q- [V^1- Q]_x- \}_y$- dargestellten linearen Polysiloxancopolymere sind dadurch gekennzeichnet, dass sie im wesentlichen keine miteinander verknüpften- $V^2$- Q- Einheiten aufweisen, oder mit anderen Worten, sind zwei- $V^2$- Q- Einheiten stets durch mindestens eine- $V^1$- Q- Einheit unterbrochen.

Bei der unregelmäßigen Verbindung, bei der beispielsweise Q- Einheiten entsprechende Monomere mit $V^1$ entsprechenden Monomer- Einheiten und $V^2$ entsprechenden Monomer- Einheiten im Verhältnis $Q/ (V^1 + V^2)$ , mit beispielsweise $V^2/V^1 > 1$ umgesetzt wird, lassen sich die linearen Polysiloxancopolymere wie folgt darstellen:

$$- Q- (V^1, V^2)-,$$

worin in V das Verhältnis $V^2/V^1$ dann > 1 ist. Dabei sind die Gruppen $V^1$ und $V^2$ statistisch über die Copolymerkette verteilt. Im Unterschied zu dem durch die regelmäßige Verbindung hergestellten linearen Polysiloxancopolymere kann dieses Copolymer auch benachbarte- Q- $V^2$- Einheiten aufweisen.

In einer bevorzugten Ausführungsform der erfindungsgemäß verwendeten Polysiloxanverbindung der Formel (I) bzw. (I') wird die Gruppe $V^1$ ausgewählt aus zweiwertigen, geradkettigen, cyclischen oder verzweigten, gesättigten, ungesättigten oder aromatischen Kohlenwasserstoffresten mit bis zu 600 bevorzugt bis zu 400 Kohlenstoffatomen, die gegebenenfalls eine oder mehrere Gruppen, ausgewählt aus -O-, -CONH-,

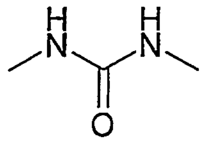 , 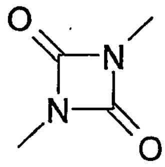 ,

$-CONR^2-$, worin $R^2$ wie oben definiert ist, , -C(O)-, -C(S)- und $Z^1$- enthalten kann, worin $-Z^1-$ eine Gruppe der Formel

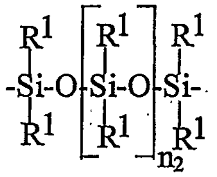

ist, worin
$R^1$ $C_1$-$C_{18}$ Alkyl, das gegebenenfalls mit einem oder mehreren Fluoratomen substituiert sein kann, oder Phenyl ist, und $n_2$ wie oben definiert ist.

In einer weiteren bevorzugten Ausführungsform der Polysiloxan-Verbindungen der Formel (I) bzw. (I') wird die Gruppe Q ausgewählt aus:

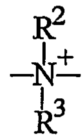

worin $R^2$ bevorzugt H oder Alkyl, bevorzugt mit 1 bis 6 Kohlenstoffatomen, ist und $R^3$ bevorzugt H, Alkyl, bevorzugt mit 1 bis 6 Kohlenstoffatomen, oder $R^O$ ist.

Bevorzugt sind in den Formeln (I) und (I'):

$R^1 = C_1$ bis $C_{18}$ Alkyl, insbesondere Methyl, Ethyl, Trifluorpropyl und Phenyl,

$n_1$ = 20 bis 400, besonders bevorzugt 20 bis 300, speziell 20 bis 200. In einer weiteren bevorzugten Ausführungsform ist $n_1$ zwischen 20 und 50 oder zwischen 80 und 200. Die Zahl $n_1$ ist die mittlere Polymerisationsgrad aus $M_n$ der Diorganosiloxy-Einheiten in der Gruppe $Z^2$.

$n_2$ = 0 bis 15, besonders bevorzugt 0 bis 10, speziell 0 bis 5, spezieller 0. Die Zahl $n_2$ ist die mittlere Polymerisationsgrad aus $M_n$ der Diorganosiloxy-Einheiten in der Gruppe $Z^1$.

Besonders bevorzugt steht

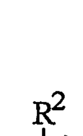

für- $NH_2^+$-, -N $(CH_3)_2^+$-, $-(NHR^O)^+$- .

$V^{2*}$ für einen zweiwertigen geradkettigen, cyclischen oder verzweigten, gesättigten, ungesättigten oder aromatischen Kohlenwasserstoffrest mit bis zu 16 Kohlenstoffatomen, der eine oder mehrere Gruppen, ausgewählt aus-O-, -CONH-, $-CONR^2-$, worin $R^2$ wie oben definiert ist, , -C (O)-, -C (S)- enthalten kann und mit einer oder mehreren Hydroxylgruppen substituiert sein kann. Noch bevorzugter wird $V^{2*}$- ausgewählt aus Gruppen der Formeln:

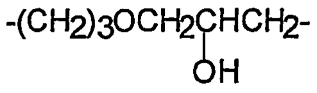 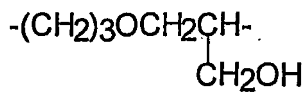

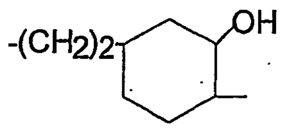 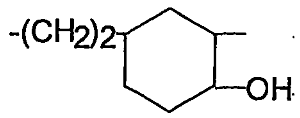

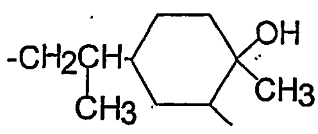 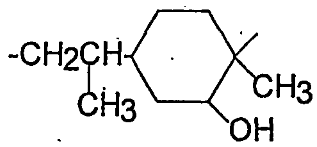

- $(CH_2)_2$-, $-(CH_2)_3$-, $-(CH_2)_4$-, $-(CH_2)_5$-, $-(CH_2)_6$-,
- $CH=CHCH_2$-, $-CH=CHCH_2CH_2$-,
- $CH_2CH_2CH_2OC(O)CH_2$-, $-CH_2CH_2CH_2OC(O)CH_2CH_2$-,
- $CH=CHCH_2OC(O)CH_2$-, $-CH=CHCH_2OC(O)CH_2CH_2$-,

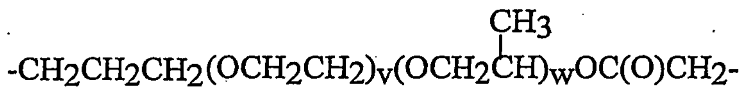

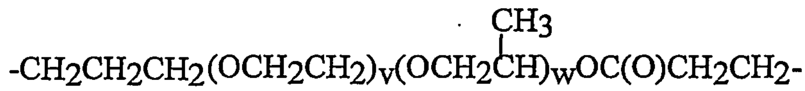

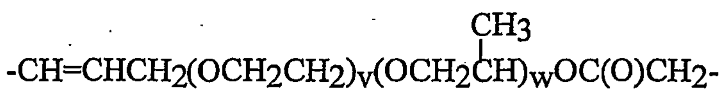

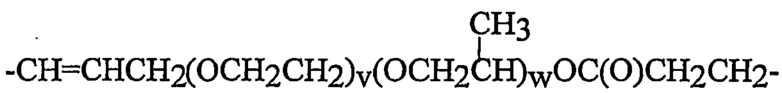

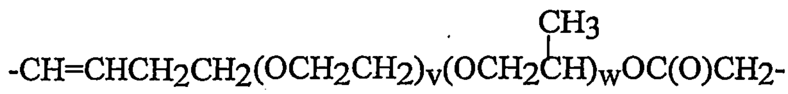

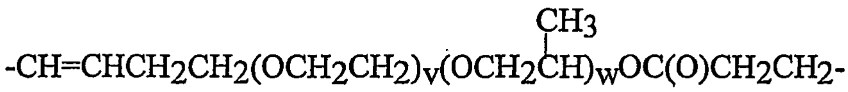

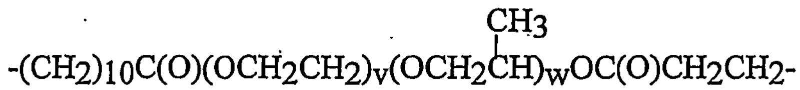

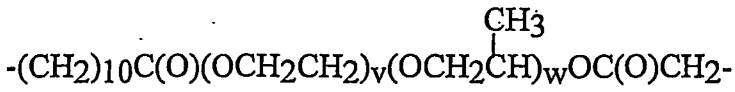

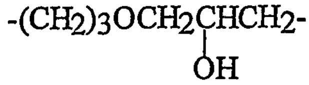
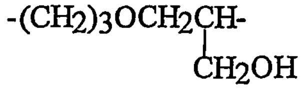

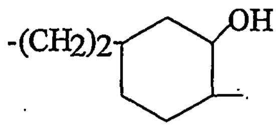
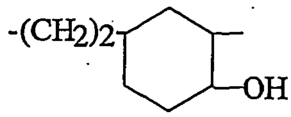

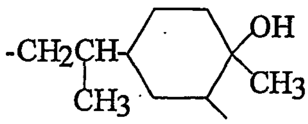
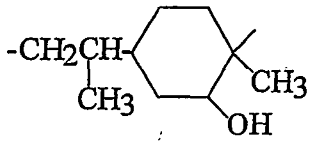

mit V+W≥ 0,
- $(CH_2)_3$-, -$(CH_2)_4$-, -$(CH_2)_5$-, -$(CH_2)_6$-,
- $CH{=}CHCH_2$-, -$CH{=}CHCH_2CH_2$-,
- $CH_2CH_2CH_2OC$ (O) $CH_2$-, -$CH_2CH_2CH_2OC$ (O) $CH_2CH_2$- .

$V^1$ steht bevorzugt für

- -$R^9$-, worin $R^9$ einen zweiwertigen, gesättigten oder einfach oder mehrfach ungesättigten, geradkettigen oder verzweigten Kohlenwasserstoffrest mit zwei bis 25 Kohlenstoffatomen darstellt,
- - $(CH_2)_uC$ (O) O- $[(CH_2CH_2O)_q$- $(CH_2CH\ (CH_3)\ O)\ _r]$- C (O) $(CH_2)_u$-
- - $(CH_2)_uC$ (O) O- $R^9$- O- C (O) $(CH_2)_u$-, worin $R^9$ wie zuvor definiert ist,
- - $(CH_2)_u$- $R^{10}$- $(CH_2)_u$-, worin $R^{10}$ eine aromatische Gruppe ist,
- - $[CH_2CH_2O]_q$- $[CH_2CH\ (CH_3)\ O]_r$- $CH_2CH_2$-,
- - CH $(CH_3)$ $CH_2O$ $[CH_2CH_2O]_q$- $[CH_2CH\ (CH_3)\ O]_r$- $CH_2CH$ $(CH_3)$-
- - $CH_2CH$ (OH) $CH_2$-,
- - $CH_2CH$ (OH) $(CH_2)_2CH$ (OH) $CH_2$-,
- - $CH_2CH$ (OH) $CH_2OCH_2CH$ (OH) $CH_2OCH_2CH$ (OH) $CH_2$- und
- - $CH_2CH$ (OH) $CH_2O$- $[CH_2CH_2O]_q$- $[CH_2CH\ (CH_3)\ O]_r$- $CH_2CH$ (OH) $CH_2$-

worin
u von 1 bis 3 ist,
q und r von 0 bis 200, bevorzugt von 0 bis 100, bevorzugter von 0 bis 70 und besonders bevorzugt 0 bis 40 ist, und $q + r > 0$ ist.

Bevorzugte Varianten von $V^1$ sind Strukturen der Formel:

- $CH_2C$ (O) O- $[CH_2CH_2O]_q$ $[CH_2CH\ (CH_3)\ O]_r$- C (O) $CH_2$-,
- $CH_2CH_2C$ (O) O- $[CH_2CH_2O]_q$- $[CH_2CH\ (CH_3)\ O]_r$- C (O) $CH_2CH_2$-,
- $CH_2CH_2CH_2C$ (O) O- $[CH_2CH_2O]_q$- $[CH_2CH\ (CH_3)\ O]_r$- C (O) $CH_2CH_2CH_2$-,
veresterte Alkylen-, Alkenylen, Alkinyleneinheiten, speziell der Strukturen
- $CH_2C$ (O) O- $[CH_2]_o$- OC (O) $CH_2$-,
- $CH_2CH_2C$ (O) O- $[CH_2]_o$- OC (O) $CH_2CH_2$-,
- $CH_2CH_2CH_2C$ (O) O- $[CH_2]_o$- OC (O) $CH_2CH_2CH_2$-
- $CH_2C$ (O) O- $CH_2C{\equiv}CCH_2$- OC (O) $CH_2$-,
- $CH_2CH_2C$ (O) O- $CH_2C{\equiv}CCH_2$- OC (O) $CH_2CH_2$-,
- $CH_2CH_2CH_2C$ (O) O- $CH_2C{\equiv}CCH_2$- OC (O) $CH_2CH_2CH_2$-,

- $CH_2C(O)O-CH_2CH=CHCH_2-OC(O)CH_2-$,
- $CH_2CH_2C(O)O-CH_2CH=CHCH_2-OC(O)CH_2CH_2-$,
- $CH_2CH_2CH_2C(O)O-CH_2CH=CHCH_2-OC(O)CH_2CH_2CH_2-$,
Alkylen-, Alkenyle-, Alkinylen- und Aryleinheiten, speziell der Strukturen: $-[CH_2]_o-$ mit o = 2 bis 6,
- $CH_2C{\equiv}CCH_2-$, $-CH_2CH=CHCH_2-$, $-CH(CH_3)CH_2CH_2-$,

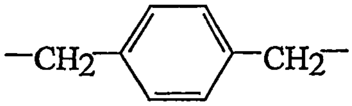

Polyalkylenoxideinheiten, speziell der Strukturen
- $[CH_2CH_2O]_q-[CH_2CH(CH_3)O]_r-CH_2CH_2-$, $-CH(CH_3)CH_2O[CH_2CH_2O]_q-[CH_2CH(CH_3)O]_r-CH_2CH(CH_3)-$
mit
mono-, di- öder polyhydroxyfunktionnelle Einheiten, speziell der Strukturen
- $CH_2CH(OH)CH_2-$, $-CH_2CH(OH)(CH_2)_2CH(OH)CH_2-$,
- $CH_2CH(OH)CH_2OCH_2CH(OH)CH_2OCH_2CH(OH)CH_2-$,
- $CH_2CH(OH)CH_2O-(CH_2CH_2O)_q-[CH_2CH(CH_3)O]_r-CH_2CH(OH)CH_2-$
mit
q =0 bis 200,
r =0 bis 200

Bevorzugt sind q = 1 bis 50, insbesondere 2 bis 50, speziell 1 bis 20, ganz speziell 1 bis 10, sowie 1 oder 2, r = 0 bis 100, insbesondere 0 bis 50, speziell 0 bis 20, ganz speziell 0 bis 10, sowie 0 oder 1 oder 2.

Die erfindungsgemäßen Polysiloxane können beispielsweise durch ein Verfahren hergestellt werden, worin

a) Ein primäres Amin, dass eine Polyalkylenoxidgruppe enthält, sowie gegebenenfalls weitere Aminverbindungen mit aminoreaktiven Polysiloxan-enthaltenden Verbindungen und gegebenenfalls weiteren aminoreaktiven Verbindungen umgesetzt werden, oder
b) Die Aminogruppe einer Polyamino- Polysiloxan- Copolymer- (Ausgangs- ) Verbindung mit einem Alkylierungsmittel, dass eine Polyalkylenoxidgruppe enthält, alkyliert wird.

Durch geeignete Auswahl der Stöchiometrie der Ausgangsverbindungen lässt sich das Verhältnis der Gruppen $V^1$, $V^2$, $R^O$ in den erfindungsgemäßen Verbindungen steuern.

Geeignete $V^1$ einführende Monomere sind beispielsweise alpha, omega- Diamine mit innenständigen Einheiten $V^1$, wie Alkylendiamine oder Diaminopolyether. Diese werden beispielsweise mit aminoreaktiven $V^2$- und/ oder $R^O$-Gruppen enthaltenden Monomeren umgesetzt, wie beispielsweise Diepoxy- Polysiloxan- Verbindungen, Dihalogenalkyl-Polysiloxanverbindungen, Mono- Amino- Polyether. Alternativ kann $V^1$ auch über Dihalogenalkyl- Verbindungen, Diepoxid- Verbindungen oder Verbindungen mir gemischten Gruppen eingeführt werden, die mit aminofunktionellen Monomeren umgesetzt werden, die die Gruppen $V^2$, $R^O$ oder weitere Gruppen $V^1$ einführen.

Zur Herstellung kann beispielsweise auf die WO 02/10257 verwiesen werden.

Die erfindungsgemäßen Polysiloxane der allgemeinen Formel (I) können Verzweigungseinheiten $V^3$ enthalten. Dabei handelt es sich um $V^3$ einen drei- oder höherwertigen, geradkettigen, cyclischen oder verzweigten, gesättigten, ungesättigten oder aromatischen Kohlenwasserstoffrest mit bis zu 1000 Kohlenstoffatomen darstellt, der gegebenenfalls eine oder mehrere Gruppen, ausgewählt aus - O-, -CONH-, $-CONR^2-$, worin $R^2$ wie oben definiert ist, -C(O)-, -C(S)-, $-Z^1-$, das wie oben definiert ist, $-Z^2-$ das wie oben definiert ist, und $Z^3$, worin $Z^3$ eine drei- oder höherwertige Organopolysiloxaneinheit ist, enthalten kann. Die Verzweigungseinheit $V^3$ kann silikonfrei sein. Beispiele hiervon schließen ein:

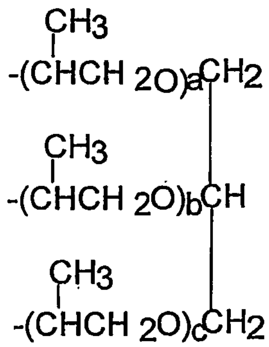

worin a, b und c gleich oder verschieden und von 1 bis 40 sein können,

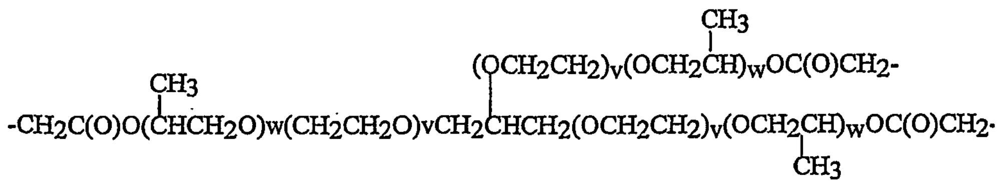

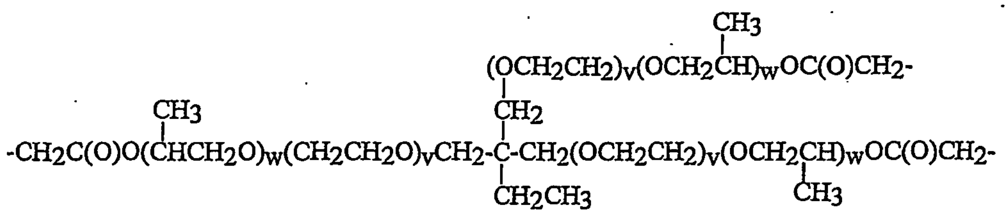

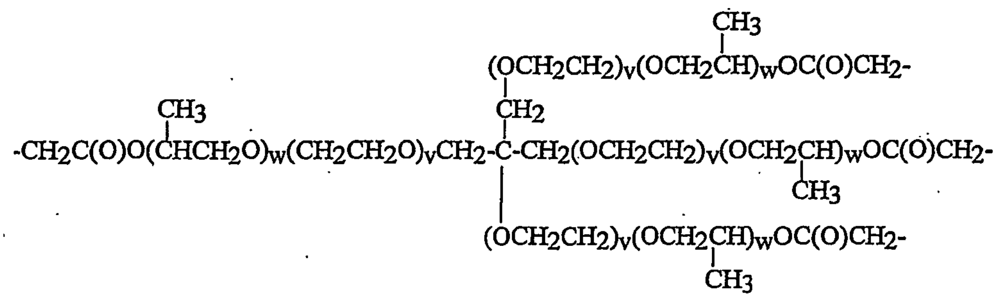

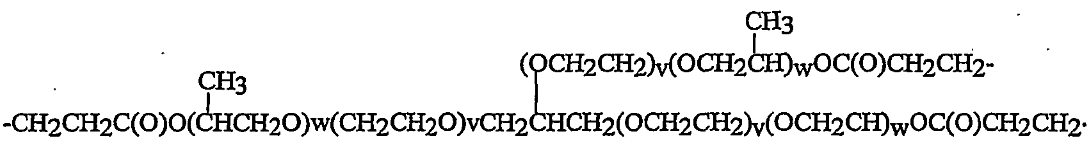

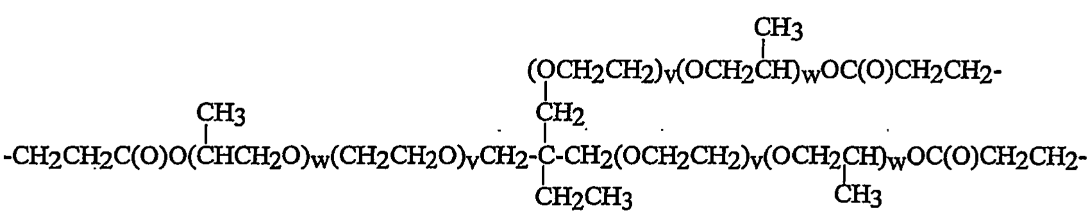

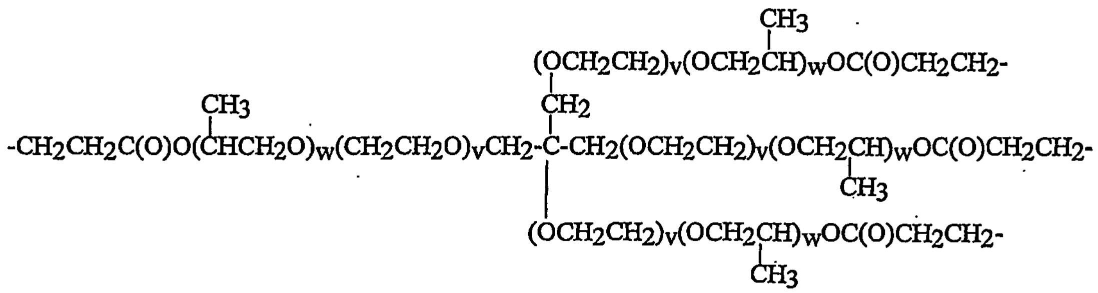

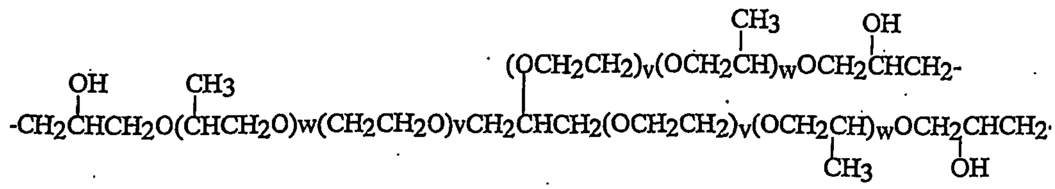

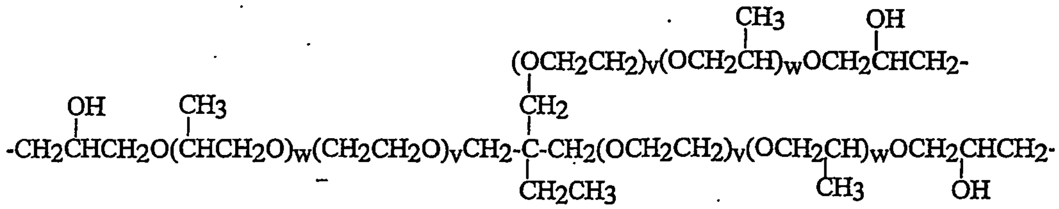

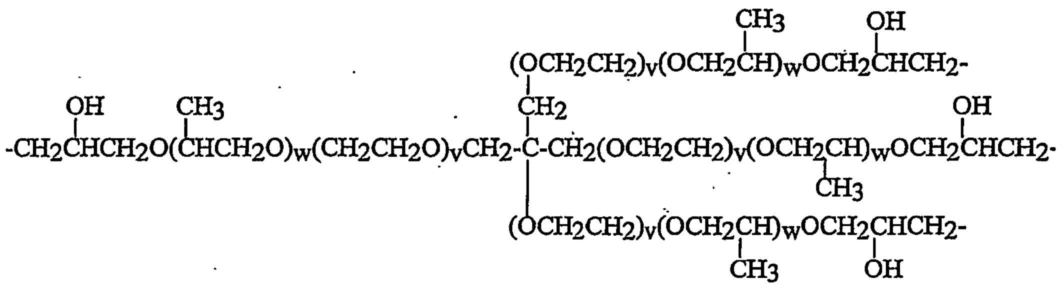

mit v+w≥ 0, wobei die Anordung der Ethylen- und Propylenoxid-Einheiten statistisch oder blockartig sein kann und die Anbindung an Q über Ethylen- und Propylenoxid-Einheiten via ein Kohlenstoffatom erfolgen kann.

Die Verzweigungseinheit $V^3$ kann eine drei- oder höherwertige Organopolysiloxaneinheit enthalten, wie zum Beispiel:

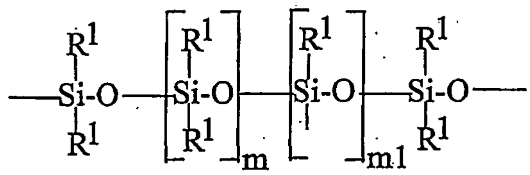

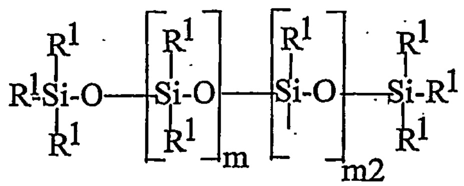

worin $R^1$ wie oben definiert ist, m = 0 bis 1000, und $m^1 \geq 1$ und $m^2 \geq 3$ ist,

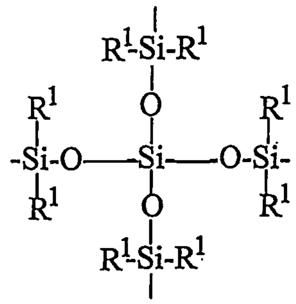

;

und

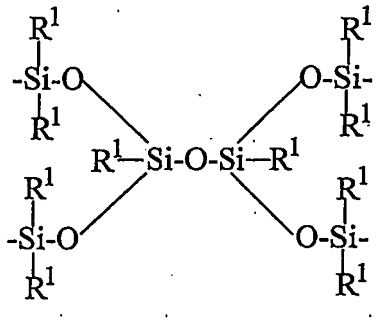

worin $R^1$ jeweils wie oben definiert ist.

Ein Beispiel einer $Z^3$-enthaltenden Verzweigungseinheit $V^3$ ist zum Beispiel:

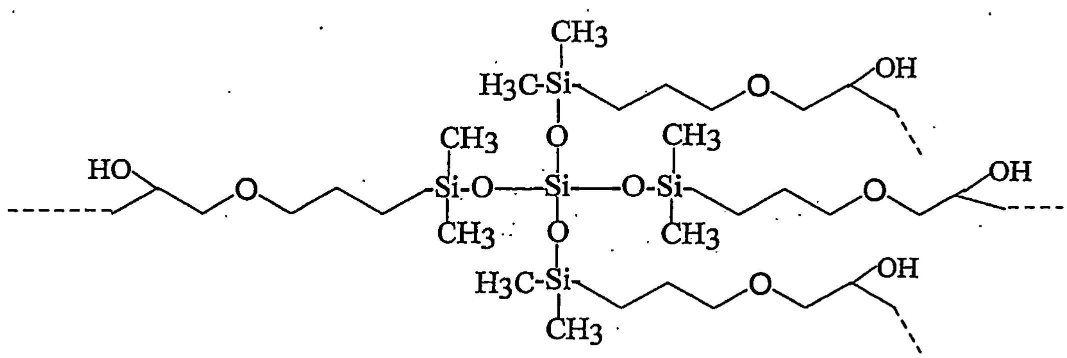

Die erfindungsgemäßen Polysiloxane enthalten die Einheiten $R^O$, die bevorzugt durch geeignete Alkylierungsreaktionen von primär, sekundär oder tertiär monoaminofunktionalisierten Polyalkylenoxiden mit reaktiv funktionalisierten Siloxanvorstufen in das Polymer eingebunden werden. Bevorzugt werden die monoprimär funktionalisierten Jeffamine® der M-Serie (Huntsman Corp.) eingesetzt.

In einer bevorzugten Ausführungsform werden die primär monoaminofunktionalisierten Polyalkylenoxide in einer vorgelagerten Reaktion zunächst mit den reaktiv funktionalisierten Siloxanen, bevorzugt Epoxysiloxanen, zu tertiären Aminen alkyliert. Diese Precursoren werden in der nachfolgenden Polymerbildungsreaktion in das Siloxanblockcopolymer eingebunden. Im Rahmen einer anderen bevorzugten Variante ist es möglich, auf diese vorgelagerte Reaktion zu verzichten und die primär monoaminofunktionalisierten Polyalkylenoxide direkt in der Polymerbildungsreaktion einzusetzen.

Für den weniger bevorzugten Fall, daß die Polyalkylenoxideinheiten die Siloxanblockpolymere gezielt terminieren sollen, kann von sekundär oder tertiär aminofunktionalisierten Polyalkylenoxideinheiten ausgegangen werden. Soweit nicht direkt verfügbar, können diese durch Vorreaktion der primär monoaminofunktionalisierten Polyalkylenoxide mit Alkylierungsmitteln, beispielsweise Monoepoxiden wie Isopropylglycidether oder Dimethylsulfat, hergestellt werden.

Das monofunktionell angebundene hydrophile Element R° wird in die erfindungsgemäßen polyquatemären Polysiloxancopolymere eingeführt, um gezielt die Hydrophilie zu steigern. Dies führt sowohl zu einer gesteigerten Hydrophilie der erfindungsgemäßen Polysiloxancopolymere selbst, so dass beispielsweise stabilere Emulsionen in Wasser entstehen, als auch zu einer Erhöhung der Hydrophilie der mit den erfindungsgemäßen Polysiloxancopolymere behandelten Substrate, was beispielsweise zu einer verbesserten Feuchtigkeitsaufnahme führt.

Es liegt im Rahmen der Erfindung, die Einführung monofunktionell angebundener hydrophiler Einheiten auf an sich bekannte alternierende Polysiloxan-Quat-Blockcopolymere anzuwenden (DE OS 3340708, US 6240929, EP 282720, DE OS 10036533).

Es liegt im Rahmen der Erfindung, die Einführung monofunktionell angebundener hydrophiler Einheiten mit der bekannten Einführung difunktioneller hydrophiler Einheiten in die Polymerhauptkette (WO 02/10257; WO 02/10259) zu kombinieren.

Es liegt weiterhin im Rahmen der Erfindung, die Einführung monofunktionell angebundener hydrophiler Einheiten mit der ebenfalls bekannten Einführung tri- und höherfunktioneller Einheiten in die Polymerhauptkette (WO 03/078504) zu kombinieren.

Es liegt weiterhin im Rahmen der Erfindung, die Einführung monofunktionell angebundener hydrophiler Einheiten mit der ebenfalls bekannten Einführung hoch geladener, oligomerisierter Quatstrukturen in die Polymer-Hauptkette (WO 2004/041912, WO 2004/042136) zu kombinieren.

Es liegt weiterhin im Rahmen der Erfindung, die Einführung monofunktionell angebundener hydrophiler Einheiten mit der ebenfalls beschriebenen Einführung reaktiver Einheiten in die Polymerhauptkette (Anmeldung PCT/EP 2004/050472) zu kombinieren. Solche reaktive Gruppen schließen Gruppen der folgenden Formeln ein:

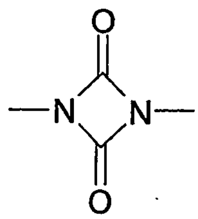

und

- Si $(OR)_{3-a}$ $(R')_a$

worin 'a' eine ganze Zahl von 0 bis 2 ist und R und R' gleich oder verschieden voneinander sein können und jeweils einen organischen Rest darstellen. Die genannten reaktiven Gruppen können erfindungsgemäß über V oder Q entsprechenden Einheiten eingeführt werden, wie in der PCT/EP 2004/050472 näher beschrieben. In einer bevorzugten Ausführungsform kann die Gruppe- Si $(OR)_{3-a}$ $(R')_a$ beispielsweise über die Verwendung von primären oder sekundären Aminen der Formel $NR_2$- (C1- C12) Akylen- Si $(OR)_{3-a}$ $(R')_a$, worin R wie oben definiert ist, eingeführt werden, wie ebenfalls in der PCT/EP 2004/050472 näher erläutert. Die reaktive Gruppe- Si $(OR)_{3-a}$ $(R')_a$ befindet sich dann an den Wiederholungseinheiten Q.

Es liegt ebenfalls im Rahmen der Erfindung, die Einführung monofunktionell angebundener hydrophiler Einheiten mit mehreren der vorstehend genannten Konzepte zu kombinieren.

Die erfindungsgemäßen quaternären Ammoniumverbindungen können bei 25°C fest oder flüssig sein. Für den Fall, dass sie bei 25°C flüssig sind, liegen die Viskositäten der genannten Polysiloxane bevorzugt zwischen 500 bis 50.000.000 mPa.s bei 25°C, bevorzugt 1000 bis 2.500.000 mPa.s bei 25°C und bei einem Schergeschwindigkeitsgefälle von D= 1 $s^{-1}$.

Die Erfindung betrifft weiterhin die Verwendung der erfindungsgemäßen Verbindungen zur Faserbehandlung bzw. Faserausrüstung.

Die Erfindung betrifft weiterhin die Verwendung der erfindungsgemäßen Verbindungen zur Erstausrüstung und Behandlung von Textilien und anderen natürlichen und synthetischen faserartigen Materialien, einschließlich Papier, Haaren und Wolle.

Die erfindungsgemäßen Verbindungen eignen sich ebenfalls zur Behandlung von Textilien und anderen natürlichen und synthetischen faserartigen Materialien in Mitteln zur Faservorbehandlung und insbesondere in nichtionogene und/oder anionische Tenside enthaltenden Waschmittelformulierungen. Zu diesem Zweck können die erfindungsgemäßen Verbindungen in Waschmittel direkt eingebaut oder aber separat in den laufenden Waschprozeß dosiert werden. Im Ergebnis der Anwendung der erfindungsgemäßen Verbindungen während des Waschprozesses, wird den behandelten Substraten eine silicontypische Weichheit, verbesserte Elastizität und verringerte Knitterneigung bei Erhalt einer akzeptablen Hydrophilie verliehen.

Weiterhin können die erfindungsgemäßen Verbindungen Verwendung finden als Bestandteil separater Weichmachersysteme nach der Wäsche von Fasern und Textilien, als Bügelhilfe und Mittel zur Verhinderung bzw. Rückgängigmachung von Textilverknitterungen.

Die Erfindung betrifft weiterhin die Verwendung der erfindungsgemäßen Verbindungen zur Behandlung und Ausrüstung von harten Oberflächen, wie Glas, Keramik, Kacheln, Kunststoffoberflächen, Metalloberflächen, Lackoberflächen, speziell Automobilkarosserien, ganz speziell in Trocknerformulierungen für die maschinelle Autowäsche.

Es ist weiterhin möglich, die erfindungsgemäßen Formulierungen in kosmetische Systeme zur Behandlung von Haaren und Haut einzuführen.

Die Erfindung betrifft weiterhin wässrige Emulsion, die mindestens eine erfindungsgemäße Polyamino- und/ oder Polyammonium- Polysiloxan- Copolymer- Verbindungen, sowie gegebenenfalls ein oder mehrere Tenside sowie gegebenenfalls ein oder mehrere stickstofffreie Polysiloxanverbindungen enthalten sowie die Verwendung der genannten wässrigen Emulsionen in einer der oben genannten Anwendungen.

**Beispiele**

**Beispiel 1**

(Einführung monofunktionell angebundener hydrophiler Einheiten in Kombination mit der Einführung difunktioneller hydrophiler Einheiten)

In einem 500 ml Dreihalskolben werden 173, 7 g (30 mniol) eines Siloxanepoxides der Struktur

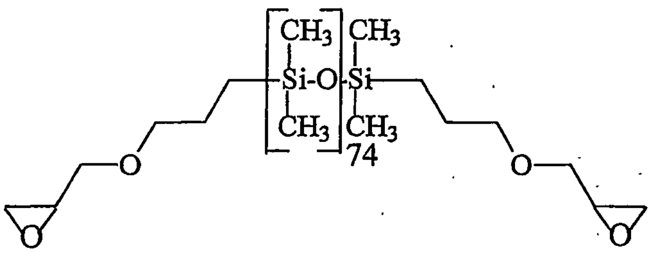

4, 17 g (4 mmol) des monofunktionellen Aminopolyethers Jeffamin® M 1000 der Struktur
$H_2N [CH (CH_3) CH_2O]_3 (CH_2CH_2O)_{19}CH_3$
und 30 ml 2- Propanol vorgelegt und unter Rührung für 6 Stunden auf 80 °C erhitzt.

In diesen Ansatz wird eine Mischung, bestehend aus
8, 51 g (4 mmol) des difunktionellen Aminopolyethers Jeffamin® ED 2003 der Struktur
$H_2NCH (CH_3) CH_2 [OCH_2CH (CH_3) ]_a (OCH_2CH_2)_{38.7} [OCH_2CH (CH_3) ]_bNH_2$
mit a+b = 6
3, 79 g (22 mmol) N, N, N', N'- Tetramethyl- 1, 6- hexandiamin
1, 68 g (28 mmol) Essigsäure
5, 6 g (28 mmol) Dodecansäure
6 ml 2- Propanol
24 ml deionisierten Wassers
gegeben.

Dieser Gesamtansatz wird für 8 Stunden auf 80 °C erhitzt, wird in Verlauf der Reaktion klar und färbt sich orange-braun. Es werden 250 g eines Polymeren mit folgenden Strukturelementen erhalten:

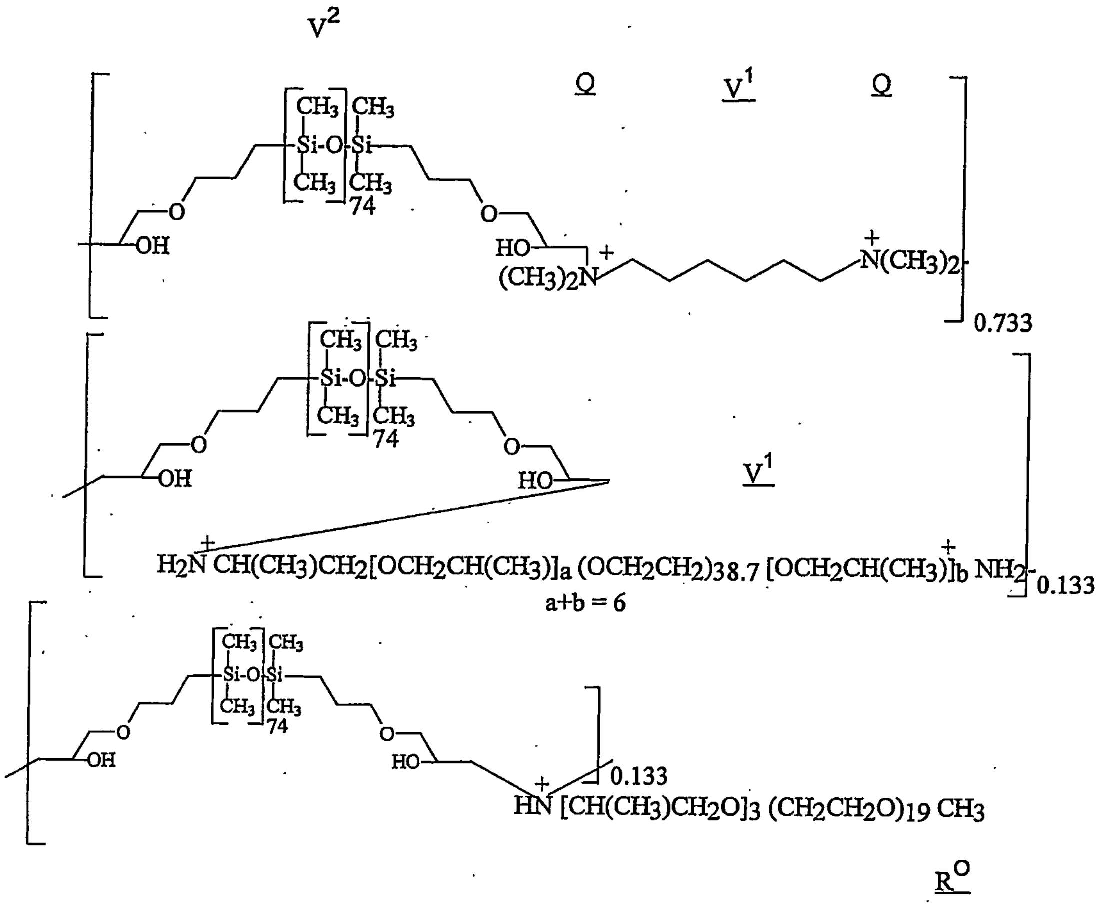

0.93 $CH_3(CH_2)_{10}COO^-$ 0.93 $CH_3COO^-$

(V1, V2, Q und $R^o$ zeigen exemplarisch die Wiederholungseinheiten bzw. Reste gemäß der Formel (I)).

**Beispiel 2**

(Einführung monofunktionell angebundener hydrophiler Einheiten in Kombination mit Polymerendstoppung)

In einem 500 ml- Dreihalskolben werden 156, 33 g (27 mmol) eines Siloxanepoxides der Struktur

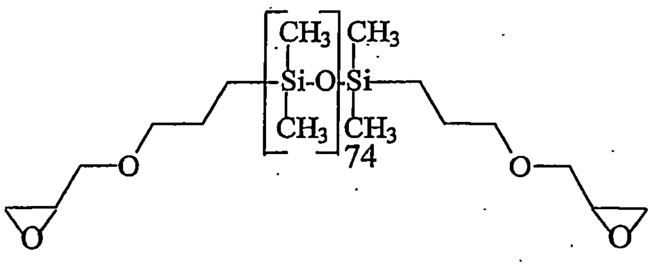

6, 25 g (6 mmol) des monofunktionellen Aminopolyethers Jeffamin® M 1000 der Struktur
$H_2N [CH (CH_3) CH_2O]_3 (CH_2CH_2O)_{19}CH_3$
und 30 ml 2- Propanol vorgelegt und unter Rührung für 6 Stunden auf 80 °C erhitzt.

Dem Ansatz werden 0, 7g (6 mmol) Isopropylglycidylether zugesetzt. Anschließend wird in diesen Ansatz eine Mischung, bestehend aus
4, 14 g (24 mmol) N, N; N', N'- Tetramethyl- 1, 6- hexandiamin
1, 62 g (27 mmol) Essigsäure

5, 4 g (27 mmol) Dodecansäure
6 ml 2- Propanol
24 ml deionisierten Wassers
gegeben.

Dieser Gesamtansatz wird für 8 Stunden auf 80 °C erhitzt. Es werden 214 g einer hellbraunen bis orange gefärbten Lösung erhalten. Das Polymer enthält folgende Strukturelemente:

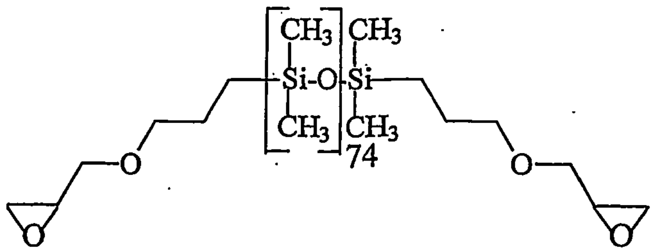

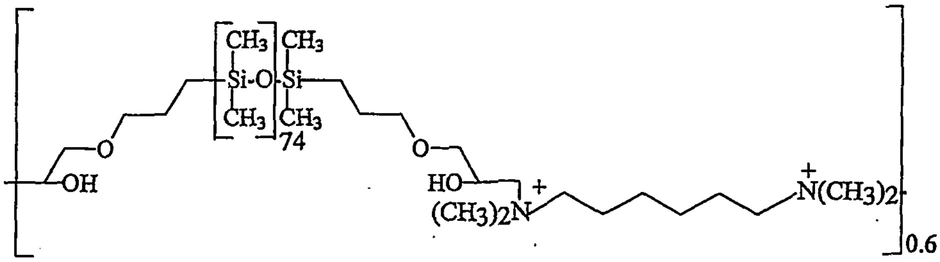

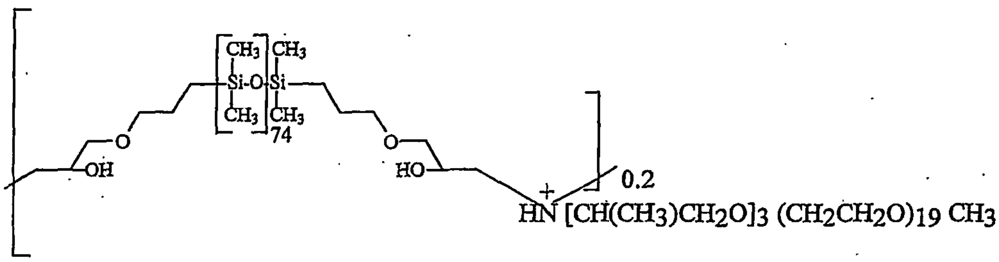

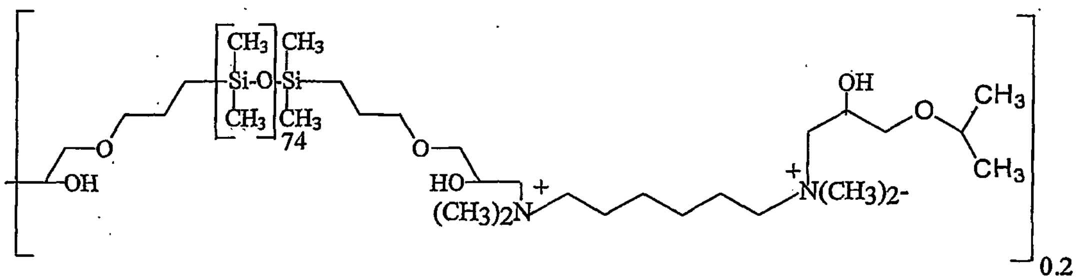

$0.9\ CH_3(CH_2)_{10}COO^-$ $0.9\ CH_3COO^-$

**Beispiel 3**

(Einführung monofunktionell angebundener hydrophiler Einheiten in Kombination mit der Einführung difunktioneller hydrophiler Einheiten und verzweigender hydrophiler Einheiten)

In einem 500 ml Dreihalskolben werden 173, 7 g (30 mmol) eines Siloxanepoxides der Struktur

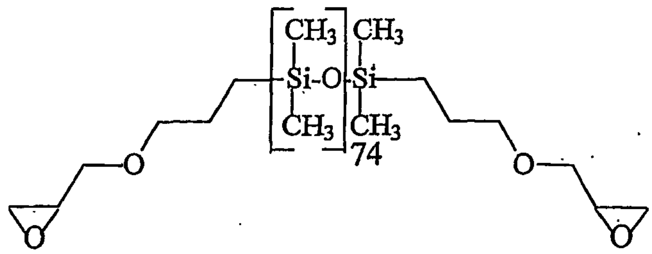

4, 17 g (4 mmol) des monofunktionellen Aminopolyethers Jeffamin® M 1000 der Struktur
$H_2N\ [CH\ (CH_3)\ CH_2O]_3\ (CH_2CH_2O)_{19}CH_3$
und 30 ml 2- Propanol vorgelegt und unter Rührung für 6 Stunden auf 80 °C erhitzt.
In diesen Ansatz wird eine Mischung, bestehend aus
6, 38 g (3 mmol) des difunktionellen Aminopolyethers Jeffamin® ED 2003 der Struktur
$H_2NCH\ (CH_3)\ CH_2\ [OCH_2CH\ (CH_3)\ ]_a\ (OCH_2CH_2)_{38.7}\ [OCH_2CH\ (CH_3)\ ]_bNH_2$
mit a+b=6
2, 1 g (0, 66 mmol) des trifunktionellen Aminopolyethers Jeffamin® T3000 der Struktur

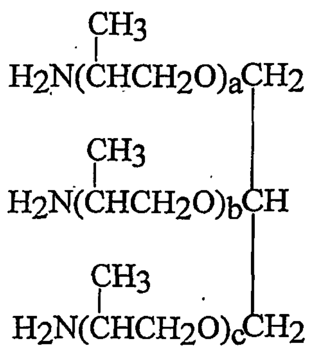

mit a+b+c = 50
3, 79 g (22 mmol) N, N, N', N'- Tetramethyl- 1, 6- hexandiamin
1, 68 g. (28 mmol) Essigsäure
5, 6 g (28 mmol) Dodecansäure
6 ml 2- Propanol
24 ml deionisierten Wassers
gegeben.

Dieser Gesamtansatz wird für 8 Stunden auf 80 °C erhitzt. Es werden 237,3 g einer orange-braunen Lösung erhalten. Das Polymer enthält folgende Strukturelemente:

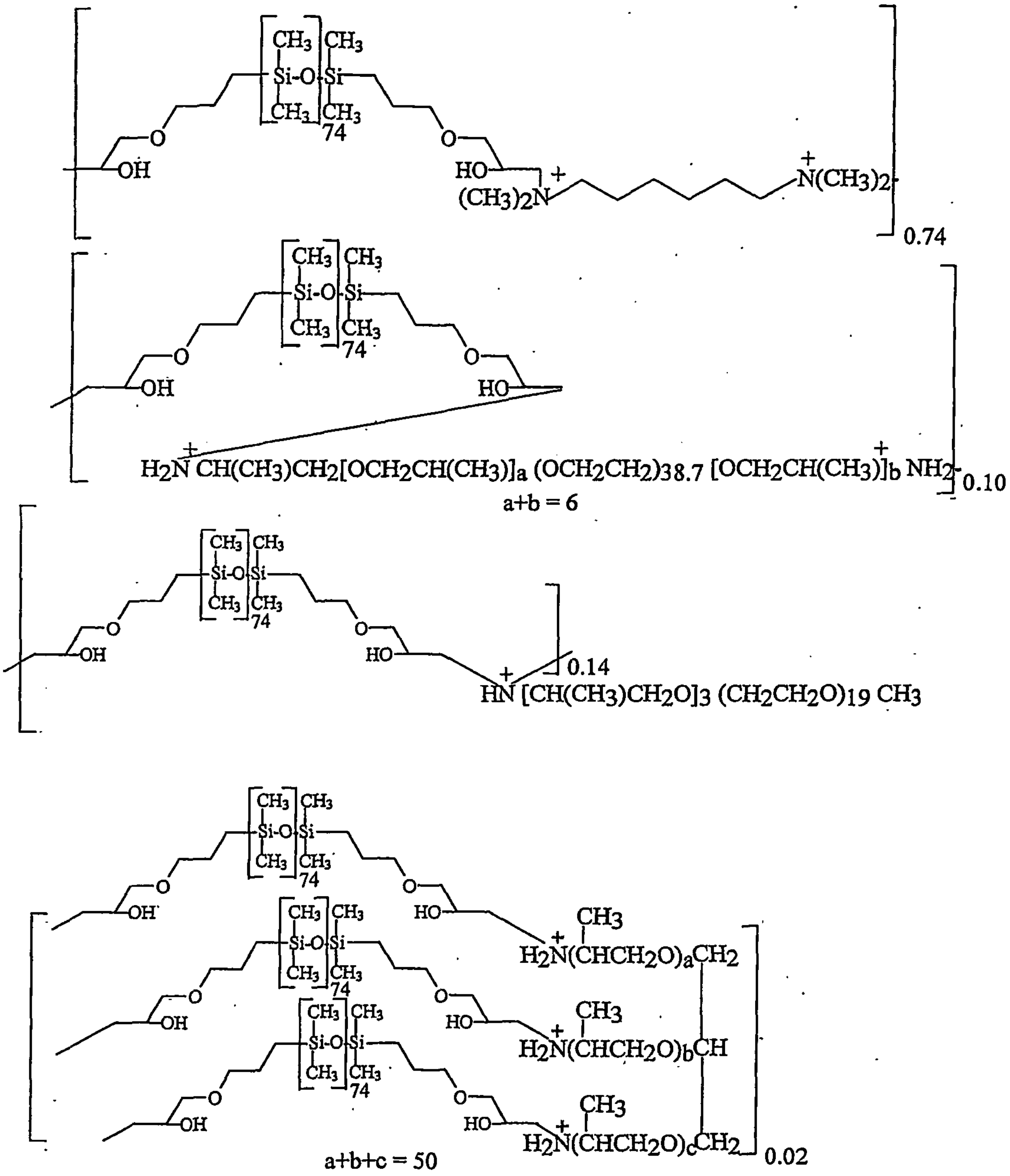

$0.94\ CH_3(CH_2)_{10}COO^-$ $0.94\ CH_3COO^-$

**Beispiel 4**

(Einführung monofunktionell angebundener hydrophiler Einheiten in Kombination mit der Einführung difunktioneller hydrophiler Einheiten und reaktiver Einheiten) 4a) Herstellung eines uretdionhaltigen ditertiären Amins

In einem 100 ml Dreihalskolben werden bei 30 bis 40 °C 4, 36 g (9, 8 mmol) Isophorondiisocyanat- Dimer der Struktur

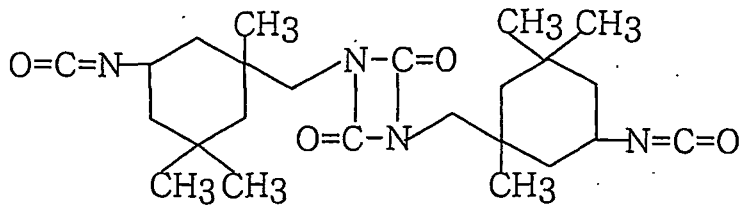

in 10, 17 g Methoxypropylacetat gelöst. Es werden unter Rührung innerhalb von 20 Minuten 2 g (19, 6 mmol) N, N-Dimethyl- 1, 3- propandiamin zugetropft, wobei die Temperatur auf 70 bis 80 °C steigt. Bei Abkühlung erfolgt Phasentrennung. Durch Zugabe von 1, 24 g 2- Propanol wird eine bei Raumtemperatur klare Lösung erhalten. Das aminomodifizierte Isophorondiisocyanat- Dimer hat die Struktur

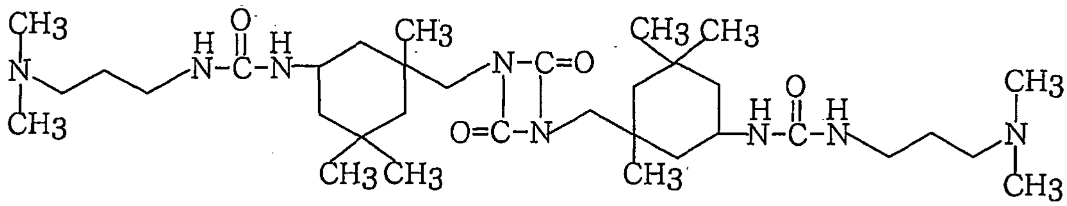

Beispiel 4b)

In einem 500 ml Dreihalskolben werden 173, 7 g (30 mmol) eines Siloxanepoxides der Struktur

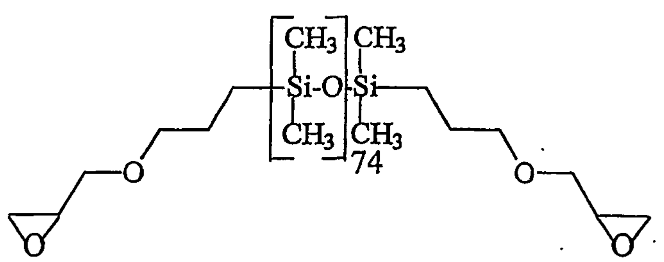

4, 17 g (4 mmol) des monofunktionellen Aminopolyethers Jeffamin® M 1000 der Struktur $H_2N\ [CH\ (CH_3)\ CH_2O]_3\ (CH_2CH_2O)_{19}CH_3$
und 30 ml 2- Propanol vorgelegt und unter Rührung für 5 Stunden auf 82- 84 °C erhitzt.

In diesen Ansatz wird eine Mischung, bestehend aus
8, 51 g (4 mmol) des difunktionellen Aminopolyethers Jeffamin® ED 2003 der Struktur
$H_2NCH\ (CH_3)\ CH_2\ [OCH_2CH\ (CH_3)\ ]_a\ (OCH_2CH_2)_{38.7}\ [OCH_2CH\ (CH_3)\ ]_bNH_2$
mit a+b=6
3, 41 g (19.8 mmol) N, N, N', N'- Tetramethyl- 1, 6- hexandiamin
1, 68 g (28 mmol) Essigsäure
5, 6 g (28 mmol) Dodecansäure
6 ml 2- Propanol
24 ml deionisierten Wassers
gegeben.

Anschließend werden dem Ansatz 4 g (2,2 mmol) des uretdionhaltigen ditertiären Amins gemäß Beispiel 4a zugesetzt und der Gesamtansatz für 8 Stunden auf 82-84 °C erhitzt. Es werden 250 g Produkt erhalten. Das Polymer enthält folgende Strukturelemente:

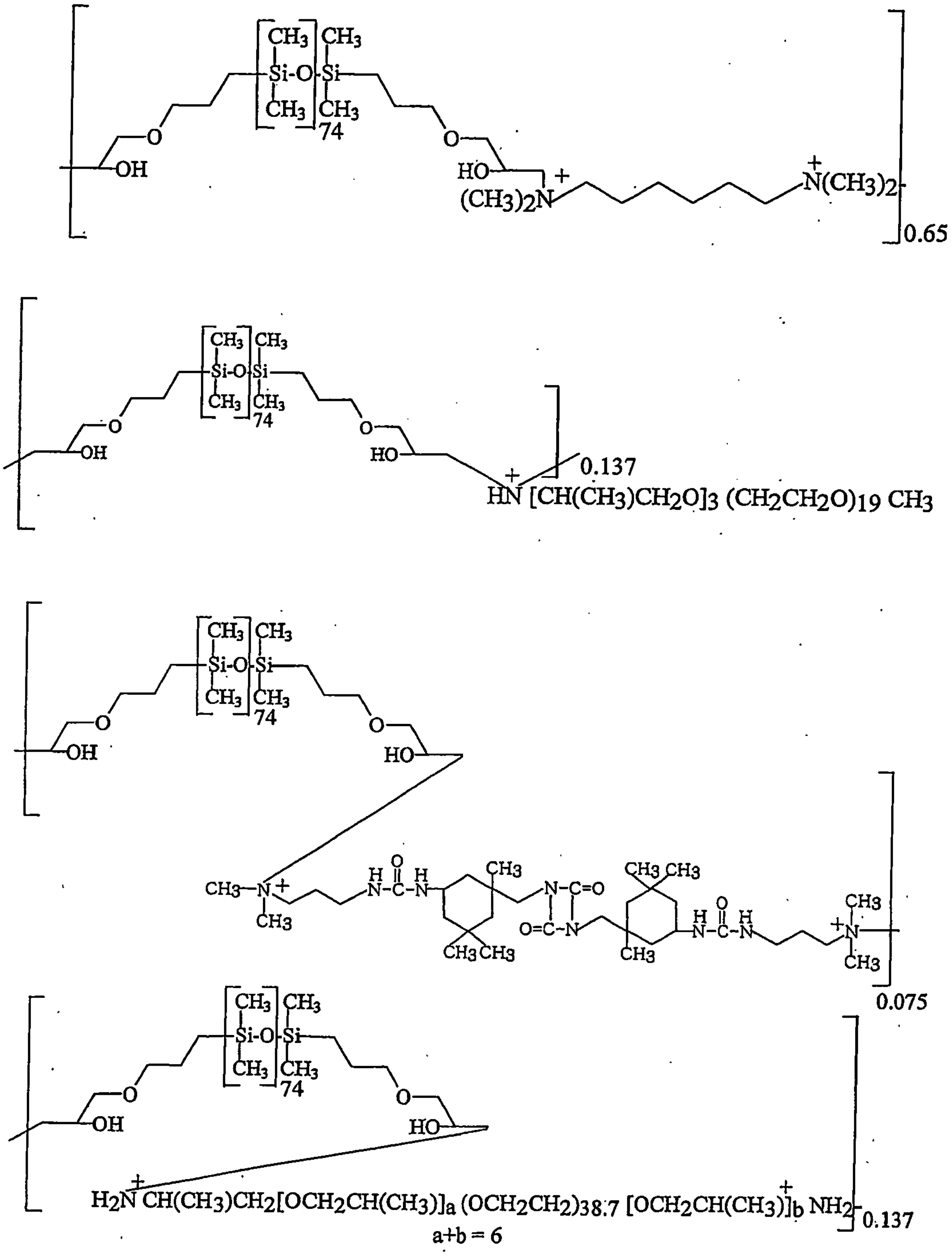

$0.93\ CH_3(CH_2)_{10}COO^-$ $0.93\ CH_3COO^-$

**Beispiel 5**

(Einführung monofunktionell angebundener hydrophiler Einheiten mit in die Kette integrierter quaternärer Ammoniumgruppe)

Beispiel 5a) Herstellung eines Chloressigsäureesters

205, 3 g (0, 5 mol) eines molmassenverteilten Octaethylenglycolmonoallylethers werden unter Stickstoff bei 20 °C Raumtemperatur vorgelegt. Unter intensiven Rühren werden innerhalb 20 Minuten 63, 4 g (0, 55 mol) Chloressigsäurechlorid zugetropft. Während des Zutropfens steigt die Temperatur auf 67 °C an und eine intensive HCl- Entwicklung setzt ein. Nach Beendigung des Zutropfens wird der Ansatz 1 Stunde auf 120 °C erhitzt. Abschließend wurden alle bis 120 °C bei 20 hPa siedenden Bestandteile abdestilliert. Es wurden 246 g eines hellgelben Esters der Struktur $ClCH_2C(O)O(CH_2CH_2O)_8CH_2CH=CH_2$ erhalten.

Beispiel 5b)

In einem 500ml Dreihalskolben werden 173,7 g (30 mmol) eines Siloxanepoxides der Struktur

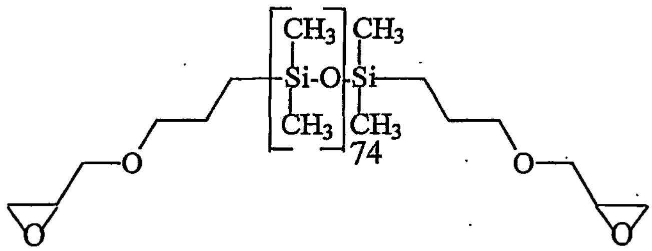

0,92 g (9 mmol) N,N-Dimethylpropylendiamin
und 100ml 2-Propanol vorgelegt und unter Rührung für 5 Stunden auf 82-84 °C erhitzt.

Anschließend werden 4,38 g (9 mmol) des Chlöressigsäureesters gemäß Beispiel 5a) zugetropft und die Mischung für weitere 5 Stunden auf auf 82-84 °C erhitzt.

In diesen Ansatz wird eine Mischung, bestehend aus
3, 62 g (21 mmol) N, N, N', N'- Tetramethyl- 1, 6- hexandiamin
1, 26 g (21 mmol) Essigsäure
4, 2 g (21 mmol) Dodecansäure
6 ml 2- Propanol
24 ml deionisierten Wassers gegeben.

Der Gesamtansatz wird für 8 Stunden auf 82-84 °C erhitzt. Es werden 361 g eines zweiphasigen Produkt erhalten. Das Polymer enthält folgende Strukturelemente:

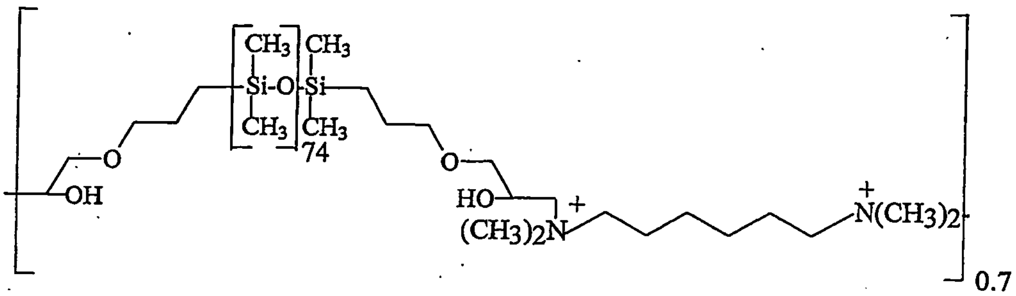

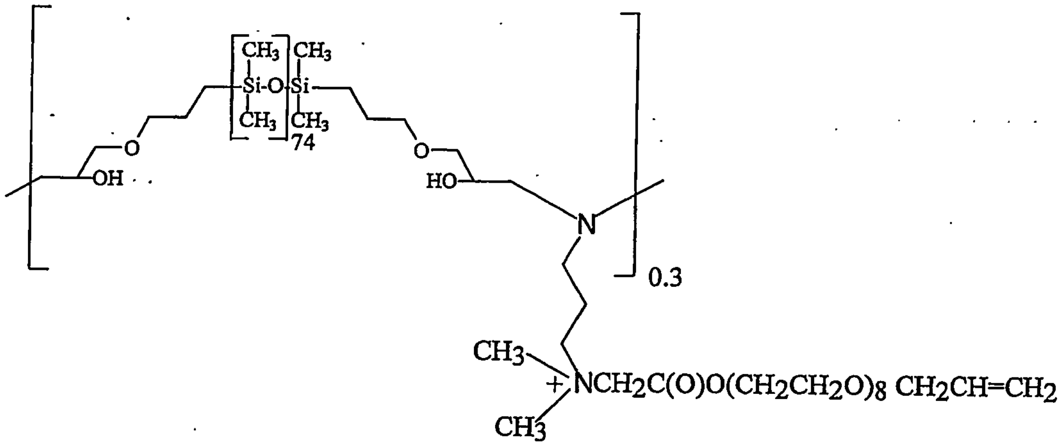

0.7 $CH_3(CH_2)_{10}COO^-$ 0.7 $CH_3COO^-$

**Beispiel 6**

(Einführung monofunktionell angebundener hydrophiler Einheiten)

In einem 500 ml Dreihalskolben werden 184, 5 g (15 mmol) eines Siloxanepoxides der Struktur

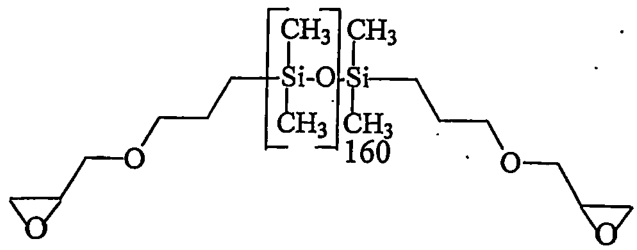

6, 25 g (3 mmol) des monofunktionellen Aminopolyethers Jeffamin® M 2070 der Struktur $H_2N$ [CH ($CH_3$) $CH_2O]_{10}$ $(CH_2CH_2O)_{32}CH_3$
und 20 g Dipropylenglycolmonobutylether vorgelegt und unter Rührung für 6 Stunden auf 100 bis 103 °C erhitzt.

In diesen Ansatz wird eine Mischung, bestehend aus
2, 07 g (12 mmol) N, N, N', N'- Tetramethyl- 1, 6- hexandiamin
0, 9 g (15 mmol) Essigsäure
3, 0 g (15 mmol) Dodecansäure
5, 5 g Dipropylenglycolmonobutylether
9, 25 ml deionisierten Wassers gegeben.

Dieser Gesamtansatz wird für 10 Stunden auf 100-103 °C erhitzt. Es werden 210 g einer 87,3-%igen Lösung eines Polymeren mit folgenden Strukturelementen erhalten:

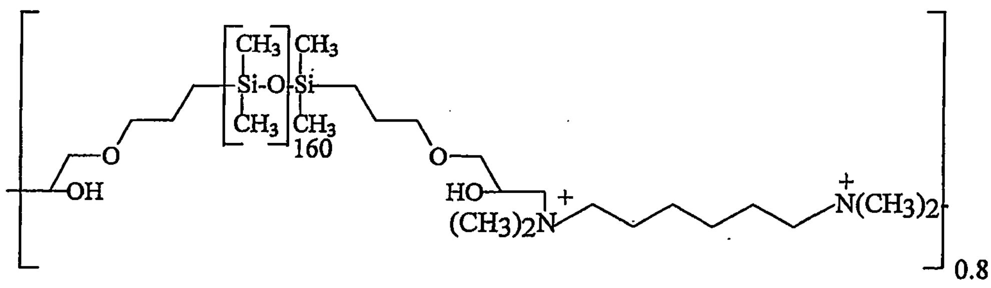

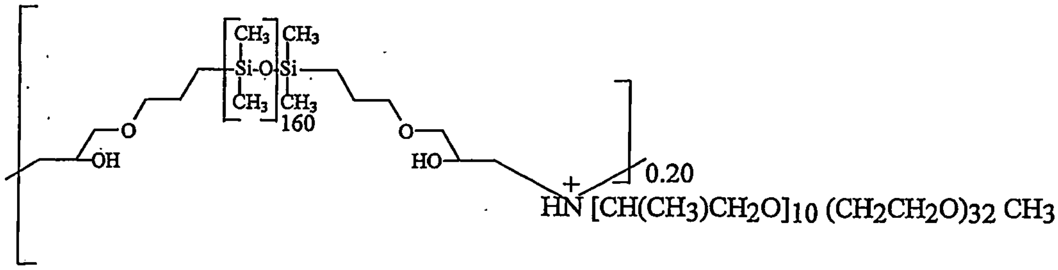

$0.9\ CH_3(CH_2)_{10}COO^-$ $0.9\ CH_3COO^-$

**Beispiel 7 nicht erfindungsgemäß**

(Einführung difunktionell eingebundener hydrophiler Einheiten)

In einem 500 ml Dreihalskolben werden 184,5 g (15 mmol) eines Siloxanepoxides der Struktur

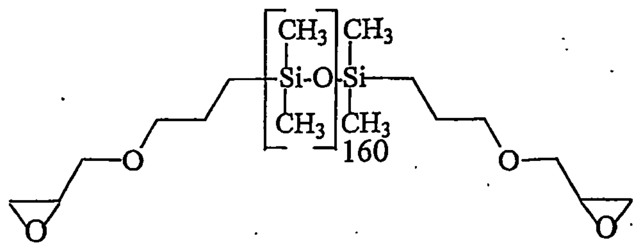

vorgelegt.

In diesen Ansatz wird eine Mischung, bestehend aus
6, 38 g (3 mmol) des difunktionellen Aminopolyethers Jeffamin® ED 2003 der Struktur
$H_2NCH\ (CH_3)\ CH_2\ [OCH_2CH\ (CH_3)\ ]_a\ (OCH_2CH_2)_{38.7}\ [OCH_2CH\ (CH_3)\ ]_bNH_2$
mit a+b=6
2, 07 g (12 mmol) N, N, N', N'- Tetramethyl- 1, 6- hexandiamin
0, 9 g (15 mmol) Essigsäure
3, 0 g (15 mmol) Dodecansäure
25, 5 g Dipropylenglycolmonobutylether
9, 25 ml deionisierten Wassers gegeben.

Dieser Gesamtansatz wird für 10 Stunden auf 100-103 °C erhitzt. Es werden 205 g einer 86 %-igen Lösung eines Polymeren mit folgenden Strukturelementen erhalten:

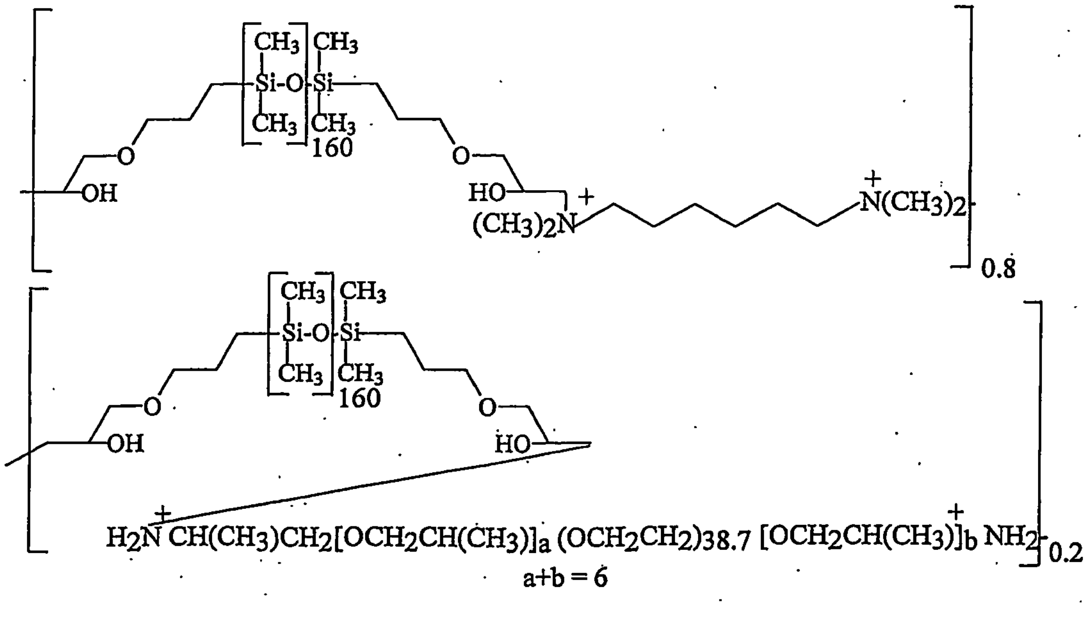

$1\ CH_3(CH_2)_{10}COO^-$ $1\ CH_3COO^-$

**Beispiel 8**

(Microemulsionen)

Es werden folgende 20 %-ige Microemulsionen hergestellt: Tab. 1

| | | Microemulsion 1 (erfindungsgemäß) | Microemulsion 2 (nicht erfindungsgemäß) |
|---|---|---|---|
| **Siloxanquatlösung (87.3%ig) gemäß Beispiel 6** | [g] | 22,9 | - |
| **Siloxanquatlösung (86%ig) gemäß Beispiel 7** | [g] | - | 23,3 |
| **Renex® 36** | [g] | 9,5 | 9,5 |
| **Renex® 30** | [g] | 2,05 | 2,05 |
| **Crodet® S40** | [g] | 1,0 | 1,0 |
| **Essigsäure** | [g] | 0,46 | 0,46 |
| **Na-Acetat** | [g] | 0,34 | 0,34 |
| **deionisiertes Wasser** | [g] | 63,7 | 63,3 |
| Renex® 36 Handelsname der ICI Surfactants; Tridecylalkohol-$EO_{12}$-OH<br>Renex® 30 Handelsname der ICI Surfactants; Tridecylalkohol-$EO_6$-OH<br>Crodet® S40 Handelsname der Croda GmbH; Stearinsäure-$EO_{40}$-OH | | | |

**Beispiel 9**

(Textilausrüstung)

Gebleichter Baumwollfrottee wird in einem Polymaten (Mathis) zur definierten Konditionierung der Textilien unter unter folgenden Randbedingungen mit den Microemulsionen 1 und 2 gemäß Beispiel 8 ausgerüstet:

Tab.2

| | |
|---|---|
| Konzentration (mg Siliconquat/g Frottee) | 15 |
| Gewicht Baumwolle (g) | 10 |
| Flottenmenge (ml) | 300 |
| Ausrüsttemperatur (°C) | 40 |
| Ausrüstzeit (min) | 30 |
| Mechanik (Zahl Stahlkugeln) | 21 |
| Trocknungstemperatur (°C) | 80 |

Zur Einstellung des Feuchtegehaltes werden die beiden Muster Baumwollfrottee für 24 Stunden unter Atmosphärenbedingungen gelagert.

Weichheit

4 Testpersonen haben den Griff der mit den Microemulsionen 1 und 2 ausgerüsteten Frotteemuster miteinander verglichen. Es konnte von keiner der Testpersonen ein signifikanter Unterschied festgestellt werden.

Hydrophilie

50 $\mu$l Wassertropfen werden auf die Frotteeoberfläche aufgesetzt und die Zeit bis zum Einsinken in Sekunden gemessen.

Tab.3

| | Tr1 | Tr2 | Tr3 | Tr4 | Tr5 | TrØ |
|---|---|---|---|---|---|---|
| Microemulsion 1 (erfindungsgemäß) | 1 | 1 | 2 | 1 | 1 | 1.2 |
| Microemulsion 2 (nicht erfindungsgemäß) | 3 | 2 | 3 | 3 | 4 | 3.0 |

Die Ergebnisse zeigen, daß für Materialien mit vergleichbarem Alkylenoxidgehalt die Hydrophilie durch gezielte Verwendung monofunktionell angebundener Alkylenoxideinheiten signifikant gesteigert werden kann, ohne daß ein merklicher negativer Einfluß auf die Griffcharakteristik ausgeübt wird.

**Patentansprüche**

1. Polyamino- Polysiloxan- und/ oder Polyammonium- Polysiloxan- Copolymer- Verbindungen, **dadurch gekennzeichnet, dass** sie Wiederholungseinheiten der Formel (I) aufweisen:

$$- [Q- V]- \qquad (I)$$

worin Q aus der Gruppe ausgewählt wird, die besteht aus:

$- NR-,$

$- N^{+}R_{2}-$

einem gesättigten oder ungesättigten diaminofunktionellen Heterocyclus der Formeln:

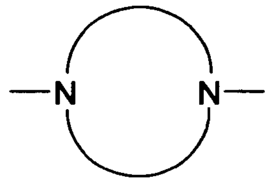

,

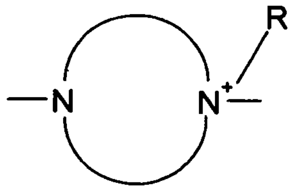

und

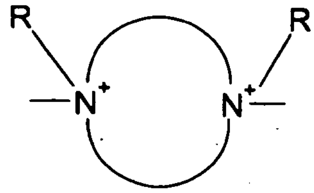

,

sowie
einem aromatischen diaminofunktionellen Heterocyclus der Formel:

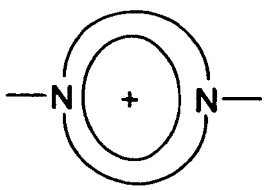

einem dreiwertigen Rest der Formel:

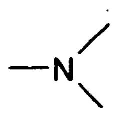

,

einem dreiwertigen Rest der Formel:

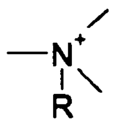

,

oder
einem vierwertigen Rest der Formel,

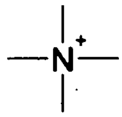

worin R jeweils Wasserstoff oder einen einwertigen organischen Rest darstellt,
wobei Q nicht an ein Carbonylkohlenstoffatom bindet,
V aus der Gruppe ausgewählt wird, die aus $V^1$, $V^2$ und $V^3$ besteht, worin
$V^2$ ausgewählt wird aus zweiwertigen, geradkettigen, cyclischen oder verzweigten, gesättigten, ungesättigten oder aromatischen Kohlenwasserstoffresten mit bis zu 1000 Kohlenstoffatomen (wobei die Kohlenstoffatome des unten definierten Polysiloxanrestes $Z^2$ nicht mitgezählt werden) , die gegebenenfalls eine oder mehrere Gruppen, ausgewählt aus
- O-, -CONH-,

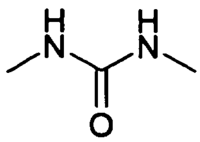 , 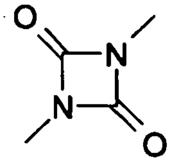 ,

- $CONR^2$-, worin $R^2$ Wasserstoff, einen einwertigen, geradkettigen, cyclischen oder verzweigten, gesättigten, ungesättigten oder aromatischen Kohlenwasserstoffrest mit bis zu 100 Kohlenstoffatomen darstellt, der eine oder mehrere Gruppen ausgewählt aus- O-, -NH-, -C (O)- und- C (S)- enthalten kann, und der gegebenenfalls durch eine oder mehrere Substituenten, ausgewählt aus der Gruppe, die besteht aus einer Hydroxylgruppe, einer gegebenenfalls substituierten, bevorzugt ein oder mehrere Stickstoffatome enthaltenden heterocyclischen Gruppe, Amino, Alkylamino, Dialkylamino, Ammonium, Polyetherresten und Polyetheresterresten substituiert sein kann, wobei wenn mehrere Gruppen- $CONR^2$ vorliegen, diese gleich oder verschieden sein können,
- C (O)- und- C (S)- enthalten kann,
der Rest $V^2$ gegebenenfalls durch eine oder mehrere Hydroxylgruppen und/ oder durch
- $Si (OR)_{3-a} (R')_a$
worin a eine ganze Zahl von 0 bis 2 ist und R und R' gleich oder verschieden voneinander sein können und jeweils einen organischen Rest darstellen, substituiert sein kann, und
der Rest $V^2$ mindestens eine Gruppe- $Z^2$- der Formel

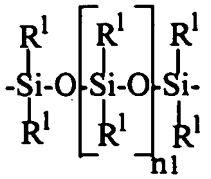

enthält, worin
$R^1$ gleich oder verschieden sein kann und aus der Gruppe ausgewählt wird, die besteht aus: $C_1$ bis $C_{22}$ Alkyl, Fluor ($C_1$- $C_{10}$) alkyl, $C_6$- $C_{10}$ Aryl und- W- $Si (OR)_{3-a} (R')_a$ besteht, worin R, R' und a wie oben definiert sind und W- O- oder einen zweiwertigen, geradkettigen, cyclischen oder verzweigten, gesättigten, ungesättigten oder aromatischen Kohlenwasserstoffrest mit bis zu 100 Kohlenstoffatomen darstellt, der eine oder mehrere Gruppen- C (O)-, -O-, -NH-, -S- enthalten kann, und gegebenenfalls durch Hydroxygruppen substituiert sein kann, und $n_1$ = 20 bis 1000 bedeutet,
$V^1$ ausgewählt wird aus zweiwertigen, geradkettigen, cyclischen oder verzweigten, gesättigten, ungesättigten oder aromatischen Kohlenwasserstoffresten mit bis zu 1000 Kohlenstoffatomen, die gegebenenfalls eine oder mehrere Gruppen, ausgewählt aus
- O-, -CONH-,

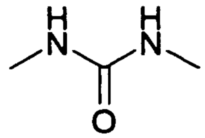, 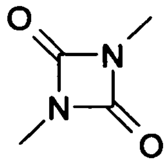,

- $CONR^2$-, worin $R^2$ wie oben definiert ist, wobei die Gruppen $R^2$ in den Gruppen $V^1$ und $V^2$ gleich oder verschieden sein können,
- C (O)-, -C (S)- und- $Z^1$- enthalten kann, worin- $Z^1$- eine Gruppe der Formel

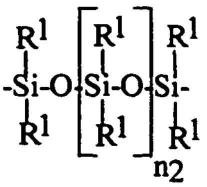

ist, worin
$R^1$ wie oben definiert ist, wobei die Gruppen $R^1$ in den Gruppen $V^1$ und $V^2$ gleich oder verschieden sein können, und $n_2$=0 bis 19 bedeutet,
und der Rest $V^1$ gegebenenfalls durch eine oder mehrere Hydroxylgruppen und/ oder durch

$$-Si(OR)_{3-a}(R')_a$$

worin a eine ganze Zahl von 0 bis 2 ist und R und R' gleich oder verschieden voneinander sein können und jeweils einen organischen Rest darstellen, substituiert sein kann, und
$V^3$ einen drei- oder höherwertigen, geradkettigen, cyclischen oder verzweigten, gesättigten, ungesättigten oder aromatischen Kohlenwasserstoffrest mit bis zu 1000 Kohlenstoffatomen darstellt, der gegebenenfalls eine oder mehrere Gruppen, ausgewählt aus
- O-, -CONH-,

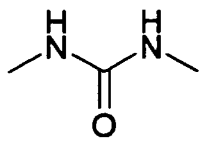, 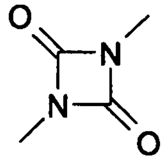, -

- $CONR^2$-, worin $R^2$ wie oben definiert ist, -C (O)-, -C (S)-, -$Z^1$-, das wie oben definiert ist, -$Z^2$- das wie oben definiert ist, und $Z^3$, worin $Z^3$ eine drei- oder höherwertige Organopolysiloxaneinheit ist, enthalten kann, und
der gegebenenfalls durch eine oder mehrere Hydroxylgruppen und/ oder durch .

$$-Si(OR)_{3-a}(R')_a$$

worin a eine ganze Zahl von 0 bis 2 ist und R und R' gleich oder verschieden voneinander sein können und jeweils einen organischen Rest darstellen, substituiert sein kann, substituiert sein kann,
mit der Maßgabe,

- dass die genannte Polysiloxan- Verbindung mindestens eine Gruppe- $Z^1$-, -$Z^2$- oder $Z^3$ enthalten,
- dass die drei- und vierwertigen Reste Q entweder der Verzweigung der aus Q und V gebildeten Hauptkette dienen, so dass die Valenzen, die nicht der Bindung in der Hauptkette dienen, weitere aus- [Q- V]- Einheiten gebildete Verzweigungen tragen, oder die drei- und vierwertigen Reste Q sättigen sich mit Resten $V^3$ innerhalb einer linearen Hauptkette ohne Bildung einer Verzweigung ab, und
- dass Gruppen Q enthalten sind, die mindestens einen Rest R aufweisen, der einen polyalkylenoxid-haltigen

organischen Rest $R^o$ darstellt,

und worin die aus Ammoniumgruppen resultierenden positiven Ladungen durch organische oder anorganische Säureanionen neutralisiert sind.

**2.** Polyamino- Polysiloxan- und/ oder Polyammonium- Polysiloxan- Copolymer- Verbindungen nach Anspruch 1, **dadurch gekennzeichnet, dass** das molare Verhältnis $R^o$ : Q von 0, 001 bis 2 beträgt.

**3.** Polyamino- Polysiloxan- und/ oder Polyammonium- Polysiloxan- Copolymer- Verbindungen nach Anspruch 1 oder 2, **dadurch gekennzeichnet, dass** $R^o$ eine Gruppe der Formel (III) darstellt,

$$- X- E- Y \qquad (III),$$

worin X eine Einfachbindung oder einen zweiwertigen, geradkettigen, verzweigten oder cyclischen Kohlenwasserstoffrest mit bis zu 20 Kohlenstoffatomen darstellt, der gegebenenfalls Stickstoff und/ oder Sauerstoff enthalten kann, und X über ein Kohlenstoffatom mit dem Stickstoffatom von Q verbunden ist,
E einen Polyalkylenoxidrest der Formel

$$- [(C_aH_{2a}) O]_y-$$

worin a = 2 bis 4 ist,
y = 2 bis 10000 ist,
darstellt, der über ein Kohlenstoffatom mit der Gruppe X und über ein Sauerstoffatom mit der Gruppe Y verbunden ist,
Y Wasserstoff oder einen einwertigen, geradkettigen, verzweigten oder cyclischen, gesättigten, ungesättigen oder aromatischen Kohlenwasserstoffrest mit bis zu 24 Kohlenstoffatomen darstellt, der Sauerstoff und/ oder Stickstoff und/ oder Halogen enthalten kann und über ein Kohlenstoffatom mit der Gruppe E verbunden ist.

**4.** Polyamino- Polysiloxan- und/ oder Polyammonium- Polysiloxan- Copolymer- Verbindungen nach einem der Ansprüche 1 bis 3, **dadurch gekennzeichnet, dass** $R^o$ eine Gruppe der Formel (III) ist, in der- E- eine Gruppe der Formel (IV) darstellt:

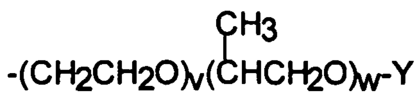

(IV),

wobei es sich um statistische und blockartige Sequenzen der Ethylen- und Propylenoxid- Einheiten handeln kann und die Bindung an E über eine Ethylen- oder Propylenoxid- Einheit erfolgen kann, mit
v = 0 bis 200,
w = 0 bis 200,
$v+w \geq 1$.

**5.** Polyamino- Polysiloxan- und/ oder Polyammonium- Polysiloxan- Copolymer- Verbindungen nach einem der Ansprüche 1 bis 4, **dadurch gekennzeichnet, dass** $R^o$ eine Gruppe der Formel (III) ist, in der Y aus H oder geradkettigen, cyclischen, verzweigten $C_1$ bis $C_{22}$- Alkyl-, Alkenyl-, Alkinyl-, Fluor- ($C_1$- $C_{10}$)- alkyl- und $C_6$- $C_{10}$- Arylresten ausgewählt wird.

**6.** Polyamino- Polysiloxan- und/ oder Polyammonium- Polysiloxan- Copolymer- Verbindungen nach einem der Ansprüche 1 bis 5, **dadurch gekennzeichnet, dass** V Wiederholungseinheiten der Formel $V^1$ und $V^2$ umfasst.

**7.** Polyamino- Polysiloxan- und/ oder Polyammonium- Polysiloxan- Copolymer- Verbindungen nach einem der Ansprüche 1 bis 6, **dadurch gekennzeichnet, dass** V polyalkylenoxid- haltige Reste umfasst.

**8.** Polyamino- Polysiloxan- und/ oder Polyammonium- Polysiloxan- Copolymer- Verbindungen nach einem der Ansprüche 1 bis 7, **dadurch gekennzeichnet, dass** das molare Verhältnis der Gruppen $V^1$ und $V^2$:

$V^2/V^1 > 1$ ist.

**9.** Polyamino- Polysiloxan- und/ oder Polyammonium- Polysiloxan- Copolymer- Verbindungen nach einem der Ansprüche 1 bis 8, **dadurch gekennzeichnet, dass** sie im Mittel mindestens zwei Wiederholungseinheiten der Formel (I) aufweisen.

**10.** Polyamino- Polysiloxan- und/ oder Polyammonium- Polysiloxan- Copolymer- Verbindungen nach einem der Ansprüche 1 bis 9, **dadurch gekennzeichnet, daß** die Gruppe $V^1$ ausgewählt wird aus zweiwertigen, geradkettigen, cyclischen oder verzweigten, gesättigten, ungesättigten oder aromatischen Kohlenwasserstoffresten mit bis zu 600 bevorzugt bis zu 400 Kohlenstoffatomen, die gegebenenfalls eine oder mehrere Gruppen, ausgewählt aus- O-, -CONH-, -$CONR^2$-, worin $R^2$ wie oben definiert ist, -C (O)-, -C (S)- und- $Z^1$- enthalten können, worin- $Z^1$- eine Gruppe der Formel

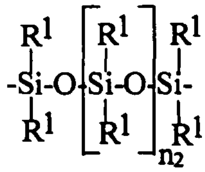

ist, worin
$R^1$ $C_1$- $C_{18}$ Alkyl, das gegebenenfalls mit einem oder mehreren Fluoratomen substituiert sein kann, oder Phenyl ist, und $n_2$ wie oben definiert ist.

**11.** Polyamino- Polysiloxan- und/ oder Polyammonium- Polysiloxan- Copolymer- Verbindungen nach einem der Ansprüche 1 bis 10, **dadurch gekennzeichnet, daß** die Gruppe $V^1$ ausgewählt wird aus:

■- $R^9$-,
worin $R^9$ einen zweiwertigen, gesättigten oder einfach oder mehrfach ungesättigten, geradkettigen oder verzweigten Kohlenwasserstoffrest mit zwei bis 25 Kohlenstoffatomen darstellt,
■- $(CH_2)_uC$ (O) O- $[(CH_2CH_2O)_q$- $(CH_2CH$ $(CH_3)$ $O)_r]$- C (O) $(CH_2)_u$-
■- $(CH_2)_uC$ (O) O- $R^9$- O- C (O) $(CH_2)_u$-, worin $R^9$ wie zuvor definiert ist,
■- $(CH_2)_u$- $R^{10}$- $(CH_2)_u$-, worin $R^{10}$ eine aromatische Gruppe ist,
■- $[CH_2CH_2O]_q$- $[CH_2CH$ $(CH_3)$ $O]_rCH_2CH_2$-,
■- CH $(CH_3)$ $CH_2O$ $[CH_2CH_2O]_q$- $[CH_2CH$ $(CH_3)$ $O]_r$- $CH_2CH$ $(CH_3)$-
■- $CH_2CH$ (OH) $CH_2$-,
■- $CH_2CH$ (OH) $(CH_2)_2CH$ (OH) $CH_2$-,
■- $CH_2CH$ (OH) $CH_2OCH_2CH$ (OH) $CH_2OCH_2CH$ (OH) $CH_2$- und
■- $CH_2CH$ (OH) $CH_2O$- $[CH_2CH_2O]_q$- $[CH_2CH$ $(CH_3)$ $O]_r$- $CH_2CH$ (OH) $CH_2$-

worin
u von 1 bis 3 ist,
q und r von 0 bis 200, bevorzugt von 0 bis 100, bevorzugter von 0 bis 70 und besonders bevorzugt 0 bis 40 ist, und $q + r > 0$ ist.

**12.** Verfahren zur Herstellung der Polyamino- Polysiloxan- und/ oder Polyammonium- Polysiloxan- Copolymer- Verbindungen nach einem der Ansprüche 1 bis 11, **dadurch gekennzeichnet, dass** man

a) Ein primäres Amin, dass eine Polyalkylenoxidgruppe enthält, sowie gegebenenfalls weitere Aminverbindungen mit aminoreaktiven Polysiloxan- enthaltenden Verbindungen und gegebenenfalls weiteren aminoreaktiven Verbindungen umsetzt, oder
b) Die Aminogruppe einer Polyamino- Polysiloxan- Copolymer- Verbindung mit einem Alkylierungsmittel, dass eine Polyalkylenoxidgruppe enthält, alkyliert.

**13.** Verwendung der Polyamino- Polysiloxan- und/ oder Polyammonium- Polysiloxan- Copolymer- Verbindungen nach einem der Ansprüche 1 bis 11 zur Faserbehandlung bzw. Faserausrüstung.

**14.** Verwendung der Polyamino- Polysiloxan- und/ oder Polyammonium- Polysiloxan- Copolymer- Verbindungen nach einem der Ansprüche 1 bis 11 zur Erstausrüstung und Behandlung von Textilien und anderen natürlichen und synthetischen faserartigen Materialien, einschließlich Papier, Haare und Wolle.

**15.** Verwendung der Polyamino- Polysiloxan- und/ oder Polyammonium- Polysiloxan- Copolymer- Verbindungen nach einem der Ansprüche 1 bis 11 zur Behandlung von Textilien und anderen natürlichen und synthetischen faserartigen Materialien in Mitteln zur Faservorbehandlung und insbesondere in nichtionogene und/ oder anionische Tenside enthaltenden Waschmittelformulierungen.

**16.** Verwendung der Polyamino- Polysiloxan- und/ oder Polyammonium- Polysiloxan- Copolymer- Verbindungen nach einem der Ansprüche 1 bis 11 als Bestandteil separater Weichmachersysteme nach der Wäsche von Fasern und Textilien, als Bügelhilfe und Mittel zur Verhinderung bzw. Rückgängigmachung von Textilverknitterungen.

**17.** Verwendung der Polyamino- Polysiloxan- und/ oder Polyammonium- Polysiloxan- Copolymer- Verbindungen nach einem der Ansprüche 1 bis 11 zur Behandlung und Ausrüstung von harten Oberflächen, wie Glas, Keramik, Kacheln, Kunststoffoberflächen, Metalloberflächen, Lackoberflächen, speziell Automobilkarosserien, ganz speziell in Trocknerformulierungen für die maschinelle Autowäsche.

**18.** Verwendung der Polyamino- Polysiloxan- und/ oder Polyammonium- Polysiloxan- Copolymer- Verbindungen nach einem der Ansprüche 1 bis 11 in kosmetischen Systemen zur Behandlung von Haaren und Haut.

**19.** Wässrige Emulsion, enthaltend mindestens eine Polyamino- Polysiloxan- und/ oder Polyammonium- Polysiloxan-Copolymer- Verbindungen nach einem der Ansprüche 1 bis 11, sowie gegebenenfalls ein oder mehrere Tenside sowie gegebenenfalls ein oder mehrere stickstofffreie Polysiloxanverbindungen.

**20.** Verwendung der wässrigen Emulsion nach Anspruch 19 in den Anwendungen, die in den Ansprüchen 13 bis 18 definiert sind.

## Claims

**1.** Polyamino- polysiloxane and/or polyammonium- polysiloxane copolymer compounds, **characterized in that** they have repeat units of the formula (I) ;

- [Q- V]- (I)

in which Q is selected from the group which consists of:

**- NR-,**

**- $N^+R_2$-**

a saturated or unsaturated diamino- functional heterocycle of the formulae:

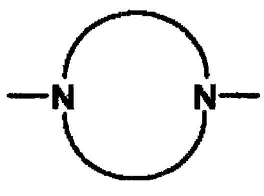

,

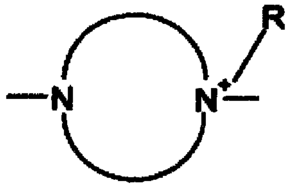

and

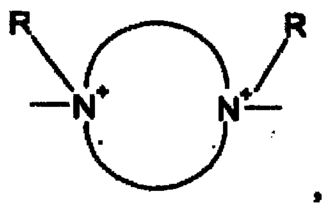

as well as
an aromatic diamino- functional heterocycle of the formula:

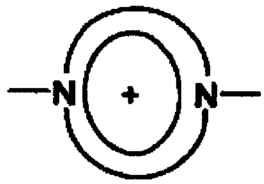

a trivalent radical of the formula:

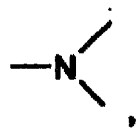

a trivalent radical of the formula:

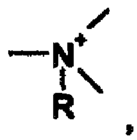

or
a tetravalent radical of the formula

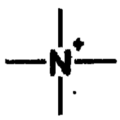

in which R is in each case hydrogen or a monovalent organic radical,
where Q does not bind to the carbonyl carbon atom,
V is selected from the group which consists of $V^1$, $V^2$ and $V^3$, In which
$V^2$ is selected from divalent straight- chain, cyclic or branched, saturated, unsaturated or aromatic hydrocarbon radicals which have up to 1000 carbon atoms (not counting the carbon atoms of the polysiloxane radical $Z^2$ defined below) and may optionally contain one or more groups selected from
**- O-, -CONH-,**

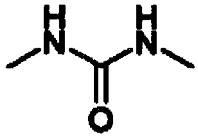 , 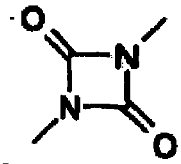 ,

- $CONR^2$-, in which $R^2$ is hydrogen, a monovalent straight- chain, cyclic or branched, saturated, unsaturated or aromatic hydrocarbon radical which has up to 100 carbon atoms, which may contain one or more groups selected from- O-, -NH-, -C (O)- and- C (S)- and may optionally be substituted by one or more substituents selected from the group which consists of a hydroxyl group, an optionally substituted heterocyclic group which preferably contains one or more nitrogen atoms, amino, alkylamino, dialkylamino, ammonium, polyether radicals and polyether ester radicals, where, when a plurality of- $CONR^2$ groups are present, they may be the same or different,
- C (O)- and- C (S)-,
the $V^2$ radical may optionally be substituted by one or more hydroxyl groups and/or by
- Si $(OR)_{3-a}$ $(R')_a$
in which a is an integer from 0 to 2 and R and R' may be the same or different from one another and are each an organic radical, and
the $V^2$ radical contains at least one- $Z^2$- group of the formula

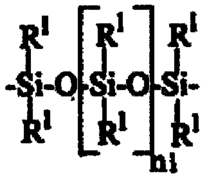

In which $R^1$ may be the same or different and is selected from the group which consists of: $C_1$- to $C_{22}$- alkyl, fluoro ($C_1$- $C_{10}$) alkyl, $C_6$- $C_{10}$- aryl and- W- Si $(OR)_{3-a}$ $(R')_a$ in which R, R' and a are each as defined above and W is- O- or a divalent straight- chain, cyclic or branched, saturated, unsaturated or aromatic hydrocarbon radical which has up to 100 carbon atoms and may contain one or more- C (O)-, -O-, -NH-, -S- groups, and may optionally be substituted by hydroxyl groups, and
$n_1$ = from 20 to 1000,
$V^1$ is selected from divalent straight- chain, cyclic or branched, saturated, unsaturated or aromatic hydrocarbon radicals which have up to 1000 carbon atoms and may optionally contain one or more groups selected from
**- O-, -CONH-,**

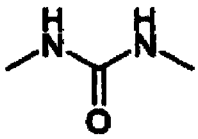 , 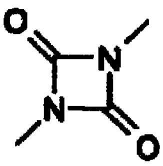 ,

- $CONR^2$-, in which $R^2$ is as defined above, where the $R^2$ groups in the $V^1$ and $V^2$ groups may be the same or different,
- C (O)-, -C (S)- and- $Z^1$- In which- $Z^1$- is a group of the formula

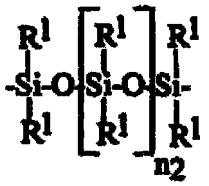

in which $R^1$ is defined above, where the $R^1$ groups in the $V^1$ and $V^2$ groups may be the same or different, and $n_2$ = from 0 to 19,
and the $V^1$ radical may optionally be substituted by one or more hydroxyl groups and/or by

$$- Si\ (OR)_{3-a}\ (R')_a$$

in which a is an integer of 0 to 2 and R and R' may be the same or different from one another and are each an organic radical, and
$V^3$ is a trivalent or higher- valency, straight- chain, cyclic or branched, saturated, unsaturated or aromatic hydrocarbon radical which has up to 1000 carbon atoms and optionally contains one or more groups selected from
**- O-, -CONH-,**

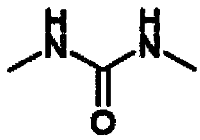
,
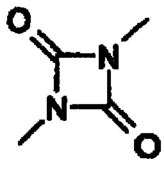
,

**- $CONR^2$-,**
in which $R^2$ is as defined above, -C (O)-, -C (S)-, -$Z^1$- which is as defined above, -$Z^2$- which is as defined above, and $Z^3$ In which $Z^3$ is a trivalent or higher- valency organopolysiloxane unit, and which may optionally be substituted by one or more hydroxyl groups and/or by

$$- Si\ (OR)_{3-a}\ (R')_a$$

in which a is an integer from 0 to 2 and R and R' may be the same or different from one another and are each an organic radical,
with the proviso

- that the polysiloxane compound mentioned contains at least one- $Z^1$-, -$Z^2$- or- $Z^3$- group,
- that the trivalent and tetravalent Q radicals either serve to branch the main chain formed from Q and V such that the valencies which do not serve for binding in the main chain bear further branches formed from - [Q-V]- units, or the trivalent and tetravalent Q radicals are saturated by $V^3$ radicals within a linear main chain without formation of a branch, and
- that there are groups Q having at least one radical R, which represent a polyalkylene oxide-containing organic radical $R^O$,

and In which the positive charges which result from ammonium groups are neutralized by organic or inorganic acid anions.

2. The polyamino-polysiloxane and/or polyammonium-polysiloxane copolymer compounds as claimed in claim 1, **characterized in that** the molar ratio of $R^o$ : Q is 0,001 to 2.

3. The polyamino- polysiloxane and/or polyammonium- polysiloxane copolymer compounds as claimed in claim 1 or 2, **characterized In that** $R^o$ is a group of the formula (III)

$$- X- E- Y \qquad (III),$$

wherein X is a single bond or a divalent straight- chain, branched or cyclic hydrocarbon radical which has up to 20 carbon atoms and may optionally contain nitrogen and/or oxygen, and X is bonded to the nitrogen atom of Q via a carbon atom,
E is a polyalkylene oxide radical of the formula

$$\mathbf{- [(C_aH_{2a})\ O]_y-}$$

In which a = 2 to 4,

y = 2 to 10000,
which is bonded to the X group via a carbon atom and to the Y group via an oxygen atom,
Y is hydrogen or a monovalent straight- chain, branched or cyclic, saturated, unsaturated or aromatic hydrocarbon radical which has up to 24 carbon atoms and may contain oxygen and/or nitrogen and/or halogen and is bonded to the E group via a carbon bond.

**4.** The polyamino-polysiloxane and/or polyammonium-polysiloxane copolymer compounds as claimed in any of claims 1 to 3, **characterized In that** the group $R^o$ is a group of the formula (III), in which -E- is a group of the formula (IV):

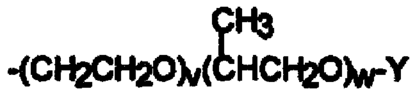

(IV),

which may comprise random and blockwise sequences of the ethylene oxide and propylene oxide units and the bond to E may be via an ethylene oxide or propylene oxide unit, where
v = from 0 to 200,
w = from 0 to 200,
$v+w \geq 1$ ,

**5.** The polyamino- polysiloxane and/or polyammonium- polysiloxane copolymer compounds as claimed in any of claims 1 to 4, **characterized In that** R° is a group of formula (III) , in which Y is selected from H or straight- chain, cyclic, branched $C_1$- to $C_{22}$- alkyl, alkenyl-, alkinyl-, fluor- ($C_1$- $C_{10}$)- alkyl and $C_6$- $C_{10}$- aryl radicals.

**6.** The polyamino-polysiloxane and/or polyammonium-polysiloxane copolymer compounds as claimed in any of claims 1 to 5, **characterized in that** V comprises repeat units of the formula $V^1$ and $V^2$.

**7.** The polyamino-polysiloxane and/or polyammonium-polysiloxane copolymer compounds as claimed in any of claims 1 to 6, **characterized in that** V comprises polyalkylene oxide-containing radicals.

**8.** The polyamino-polysiloxane and/or polyammonium-polysiloxane copolymer compounds as claimed in any of claims 1 to 7, **characterized in that** the molar ratio of the $V^1$ and $V^2$ groups is:

$V^2/V^1 > 1$.

**9.** The polyamino-polysiloxane and/or polyammonium-polysiloxane copolymer compounds as claimed In any of claims 1 to 8, **characterized In that** they have an average of at least two repeat units of the formula (I).

**10.** The polyamino- polysiloxane and/or polyammonium- polysiloxane copolymer compounds as claimed in any of claims 1 to 9, **characterized In that** the $V^1$ group is selected from divalent straight- chain, cyclic or branched, saturated, unsaturated or aromatic hydrocarbon radicals which have up to 600, preferably up to 400 carbon atoms and may optionally contain one or more groups selected from- O-, -CONH-, -$CONR^2$-, in which $R^2$ is as defined above, -C (O)- .- C (S)- and- $Z^1$-, in which- $Z^1$- is a group of the formula

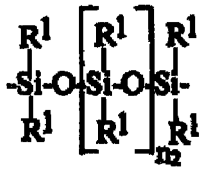

wherein $R^1$ is $C_1$- $C_{18}$- alkyl which is optionally substituted by one or more fluorine atoms, or phenyl, and $n_2$ is as defined above.

**11.** The polyamino-polysiloxane and/or polyammonium-polysiloxone copolymer compounds as claimed in any of claims 1 to 10, **characterized In that** the $V^1$ group is selected from:

- -$R^9$-,

wherein $R^9$ is a divalent saturated or mono- or polyunsaturated, straightchain or branched hydrocarbon radical which has from two to 25 carbon atoms,

- ■ **$-(CH_2)_uC(O)O-[(CH_2CH_2O)_q-(CH_2CH(CH_3)O)_r]-C(O)(CH_2)_n-$**
- ■ **$-(CH_2)_uC(O)O-R^9-O-C(O)(CH_2)_u-$,** In which $R^9$ is as defined above,
- ■ **$-(CH_2)_u-R^{10}-(CH_2)_u-$** , in which $R^{10}$ is an aromatic group,
- ■ **$-[CH_2CH_2O]_q-[CH_2CH(CH_3)O]_r-CH_2CH_2-$,**
- ■ **$-CH(CH_3)CH_2O[CH_2CH_2O]_q-[CH_2CH(CH_3)O]_{rr}-CH_2CH(CH_3)-$**
- ■ **$-CH_2CH(OH)CH_2-$,**
- ■ **$-CH_2CH(OH)(CH_2)_2CH(OH)CH_2-$,**
- ■ **$-CH_2(CH(OH)CH_2OCH_2CH(OH)CH_2OCH_2CH(OH)CH_2-$** and
- ■ **$-CH_2CH(OH)CH_2O-[CH_2CH_2O]_q-[CH_2CH(CH_3)O]_r-CH_2CH(OH)CH_2-$**

wherein
u is from 1 to 3,
q and r are from 0 to 200, preferably from 0 to 100, more preferably from 0 to 70 and still more preferably from 0 to 40, and
$q+r > 0$.

**12.** A process for preparing the polyamino-polysiloxane and/or polyammonium-polysiloxane copolymer compounds as claimed in any one of claims 1 to 11, **characterized in that**

a) a primary amine which contains a polyalkylene oxide group and optionally further amine compounds are reacted with amino-reactive polysiloxane-containing compounds and optionally further amino-reactive compounds, or
b) the amino group of a polyamino-polysiloxane copolymer compound is alkylated with an alkylating agent which contains a polyalkylene oxide group.

**13.** An use of the polyamino-polysiloxone and/or polyammonium-polysiloxane copolymer compounds as claimed in any one of claims 1 to 1 for fibre treatment or fiber finishing.

**14.** An use of the polyamino-polysiloxane and/or polyammonium-polysiloxane copolymer compounds as claimed in any one of claims 1 to 11 for the initial finishing and treatment of textiles and other natural and synthetic fibrous materials including paper, hair and wool.

**15.** An use of the polyamino-polysiloxane and/or polyammonium-polysiloxane copolymer compounds as claimed in any one of claims 1 to 11 for the treatment of textiles and other natural or synthetic fibrous materials in agents for the fibre pretreatment and in particular in detergent formulations containing non-ionogenic and/or anionic surfactants.

**16.** An use of the polyamino-polysiloxane and/or polyammonium-polysiloxane copolymer compounds as claimed In any one of claims 1 to 11 as constituent of separate softener systems after washing of fibers and textiles, as ironing aids and agent for preventing or reversing of textile creasings.

**17.** An use of the polyamino-polysiloxane and/or polyammonium-polysiloxane copolymer compounds as claimed in any one of claims 1 to 11 for the treatment and finishing of hard surfaces such as glass, ceramics, tiles, plastic surfaces, metal surfaces, varnish surfaces, in particular car bodies, more particularly in drying agent formulations for automatic car washing,

**18.** An use of the polyamino-polysiloxane and/or polyammonium-polysiloxane copolymer compounds as claimed in any one of claims 1 to 11 in cosemtic systems for the treatment of hair and skin.

**19.** An aqueous emulsion containing at least one polyamino-polysiloxane and/or polyammonium-polyslloxane copolymer compound as claimed In any one of claims 1 to 11 and optionally one or more surfactants and optionally one or more nitrogen-free polysiloxone compounds.

**20.** An use of the aqueous emulsion as claimed in claim 19 in the applications as claimed in any one of claims 13 to 18.

## Revendications

1. Composés copolymères de polyamino- polyslloxane et/ou de polyammonium- polysiloxane, **caractérisés en ce qu'**ils ont des unités répétitives de la formule (I) :

- [Q- V]- (I)

dans laquelle Q est sélectionné parmi le groupe qui consiste en:

**- NR-,**

**- $N^+R_2$-**

un hétérocycle à fonction diamine saturé ou non saturé des formules :

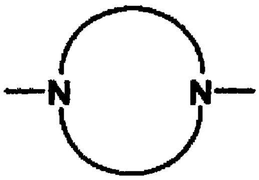

,

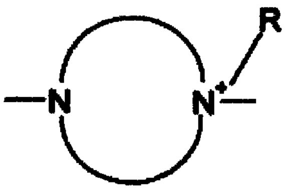

et

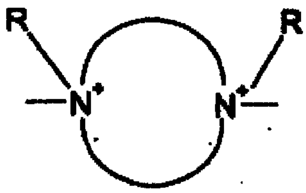

,

et

un hétérocycle à fonction diamine aromatique de la formule:

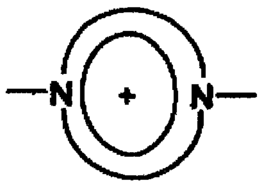

un radical trivalent de la formule :

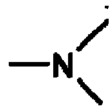,

un radical trivalent de la formule:

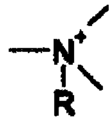,

ou

un radical tétravalent de la formule

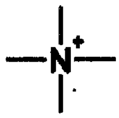

dans lesquelles chacun de R est de l'hydrogène ou un radical organique monovalent,

où Q ne lie pas à un atome de carbone carbonyle.

V est sélectionné parmi le groupe qui consiste en $V^1$, $V^2$ et $V^3$, dans lequel

$V^2$ est sélectionné parmi des radicaux d'hydrocarbure divalents, linéaires, cycliques ou ramifiés, saturés, non saturés ou aromatiques ayant jusque 1000 atomes de carbone (ne comptant pas les atomes de carbone du radical de polysiloxane $Z^2$ comme défini ci- dessous) , qui peuvent éventuellement comprendre un ou plusieurs groupes sélectionnés parmi

**- O-, -CONH-,**

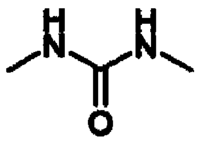, 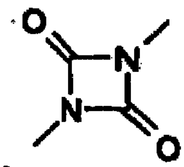,

- $CONR^2$-, dans laquelle $R^2$ représente de l'hydrogène, un radical d'hydrocarbure monovalent, linéaire, cyclique ou ramifié, saturé, non saturé ou aromatique ayant jusque 100 atomes de carbone, qui peut comprendre un ou plusieurs groupes sélectionnés parmi- O-, -NH-, -C (O)- et- C (S)- et qui peut être éventuellement substitué par un ou plusieurs substltuants sélectionnés parmi le groupe qui consiste en un groupe hydroxyle, un groupe hétérocyclique éventuellement substitué contenant de préférence un ou plusieurs atomes d'azote, amino, alkylamino, dialkylamino, ammonium, des radicaux polyéther ou des radicaux polyétherester, où, lorsqu'il y a plusieurs groupes- $CONR^2$, ils peuvent être identiques ou différents,

- C (O)- and- C (S)-,

le radical $V^2$ peut être éventuellement substitué par un ou plusieurs groupes hydroxyle et/ou par

"$Si\ (OR)_{3-a}\ (R')_a$

dans laquelle a est un nombre entier de 0 à 2 et R et R' peuvent être identiques ou différents et chacun représente un radical organique et

le radical $V^2$ contient au moins un groupe- $Z^2$- de la formule

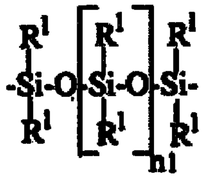

dans laquelle

$R^1$ peuvent être identiques ou différents et sont sélectionnés parmi le groupe qui consiste en : $C_1$ à $C_{22}$ alkyle, fluor ($C_1$- $C_{10}$) alkyle, $C_6$- $C_{10}$ aryle et- W- Si $(OR)_{3-a}$ $(R')_a$, dans laquelle R, R' et a sont tels que définis ci- dessus et W représente- O- ou un radical d'hydrocarbure divalent, linéaire, cyclique ou ramifié, saturé, non saturé ou aromatique ayant jusque 100 atomes de carbone qui peut contenir un ou plusieurs groupes- C (O)-, -O-, -NH-, -S- et peut être éventuellement substitué par des groupes hydroxyle et

$n_1$ = de 20 à 1000,

$V^1$ est sélectionné parmi des radicaux d'hydrocarbure divalents, linéaires, cycliques ou ramifiés, saturés, non saturés ou aromatiques ayant jusque 1000 atomes de carbone, qui peut éventuellement contenir un ou plusieurs groupes sélectionnés parmi

- O-, -CONH-,

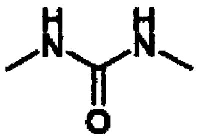 , 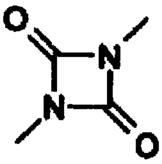 ,

- $CONR^2$-, dans laquelle $R^2$ est tel que défini ci- dessus, où les groupes $R^2$ dans des groupes $V^1$ et $V^2$ peuvent être identiques ou différents,

- C (O)-, -C (S)- et- $Z^1$-, dans lequel- $Z^1$- est un groupe de la formule

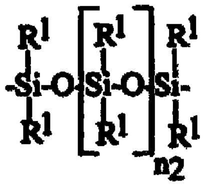

dans laquelle $R^1$ est tel que défini ci- dessus, où les groupes $R^1$ dans les groupes $V^1$ et $V^2$ peuvent être identiques ou différents et

$n_2$ = de 0 à 19,

et le radical $V^1$ peut être éventuellement substitué par un ou plusieurs groupes hydroxyle et/ou par

$$- Si (OR)_{3-a} (R')_a$$

dans laquelle a est un nombre entier de 0 à 2 et R et R' peuvent être identiques ou différents et chacun représente un radical organique et

$V^3$ représente un radical d'hydrocarbure trivalent ou d'une valence supérieure, linéaire, cyclique ou ramifié, saturé, non saturé ou aromatique ayant jusque 1000 atomes de carbone, qui peut éventuellement contenir un ou plusieurs groupes sélectionnés parmi

- O-, -CONH-,

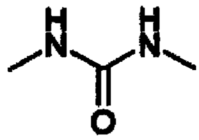 , 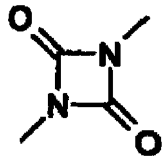 ,

- $CONR^2$-,

dans laquelle $R^2$ est tel que défini ci- dessus, C (O)-, -C (S)-, -$Z^1$- sont tels que définis ci- dessus, -$Z^2$- est tel que défini ci- dessus et $Z^3$, dans lequel $Z^3$ est une unité organopolysiloxane trivalent ou d'une valence supérieure et qui peut être éventuellement substitué par un ou plusieurs groupes hydroxyle et/ou par

$$- Si (OR)_{3-a} (R')_a$$

dans laquelle a est un nombre entier de 0 à 2 et R et R' peuvent être identiques ou différents et chacun représente un radical organique,
à la condition

- que ledit composé de polysiloxane contienne au moins un groupe- $Z^1$-, -$Z^2$- ou $Z^3$,
- que les radicaux Q trivalents ou tétravalents soit servent de la ramification de la chaîne principale formée par Q et V, afin que les valences, qui ne servent pas à la liaison dans la chaîne principale, présentent des autres ramifications formées par des unités de -[Q-V]- soit les radicaux Q trivalents ou tétravalents se saturent avec des radicaux $V^3$ dans une chaîne principale linéaire sans formant une ramification et
- que des groupes Q soient compris, qui présentent au moins un radical R, qui représente un radical $R^o$ organique contenant un oxyde de polyalkylène

et dans lesquels la charge positive résultant des groupes ammonium est neutralisée par des anions d'acide organique ou inorganique.

**2.** Composés copolymères de polyamlno-polysiloxane et/ou de polyammonium-polysiloxane selon la revendication 1, **caractérisés en ce que** le rapport molaire $R^o$ : Q est de 0,001 à 2.

**3.** Composés copolymères de polyamino- polysiloxane et/ou de polyammonium- polysiloxane selon l'une des revendications 1 ou 2, **caractérisés en ce que** $R^o$ représente un groupe de la formule (III)

$$- X- E- Y \qquad (III) ,$$

dans laquelle X est une liaison simple ou un radical d'hydrocarbure divalent, linéaire, ramifié ou cyclique ayant jusque 20 atomes de carbone, qui peut éventuellement contenir de l'azote et/ou de l'oxygène et X est lié avec l'atome de l'azote de Q par un atome de carbone,
E est un radical de l'oxyde de polyalkylène de la formule

$$- [(C_aH_{2a}) O]_y-$$

dans laquelle
a = 2 à 4,
y = 2 à 10000.
qui est lié au groupe X par un atome de carbone et au groupe Y par un atome d'oxygène,
Y représente de l'hydrogène ou un radical d'hydrocarbure monovalent, linéaire, ramifié ou cyclique, saturé, non saturé ou aromatique ayant jusque 24 atomes de carbone, qui peut contenir de l'oxygène et/ou de l'azote et/ou de l'halogène et est lié au groupe E par un atome de carbone,

**4.** Composés copolymères de polyamino-polysiloxane et/ou de polyammonium-polysiloxane selon l'une des revendications 1 à 3, **caractérisés en ce que** $R^o$ est un groupe de la formule (III), dans laquelle -E- est un groupe de la formule (IV) :

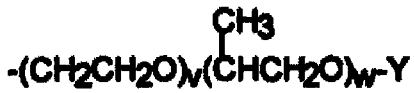

(IV),

qui peut comprendre des séquences statistiques et en block des unités de l'oxyde d'éthylène et l'oxyde de propylène et la liaison à E peut être réalisée par une unité de l'oxyde d'éthylène ou de l'oxyde de propylène, avec
v = de 0 à 200,
w = de 0 à 200,
v+w ≥ 1.

**5.** Composés copolymères de polyamino- polysiloxane et/ou de polyammonium- polysiloxane selon l'une des revendications 1 à 4, **caractérisés en ce que** $R^o$ est un groupe de la formule (III) , dans laquelle Y est sélectionné parmi H ou des radicaux $C_1$ à $C_{22}$ alkyle, alcényle, alcynyle, fluor- ($C_1$- $C_{10}$)- alkyle et $C_6$- $C_{10}$- aryle linéaires, cycliques ou ramifiés.

**6.** Composés copolymères de polyamino-polysiloxane et/ou de polyammonium-polysiloxane selon l'une des revendications 1 à 5, **caractérisés en ce que** V comprend des unités répétitives de la formule $V^1$ et $V^2$.

**7.** Composés copolymères de polyamino-polysiloxane et/ou de polyammonium-polysiloxane selon l'une des revendications 1 à 6, **caractérisés en ce que** V comprend des radicaux contenant de l'oxyde polyalkylène.

**8.** Composés copolymères de polyamino-polysiloxane et/ou de polyammonium-polysiloxane selon l'une des revendications 1 à 7, **caractérisés en ce que** le rapport molaire des groupes $V^1$ et $V^2$ est $V^2/V^1 > 1$.

**9.** Composés copolymères de polyamino-polysiloxane et/ou de polyammonium-polysiloxane selon l'une des revendications 1 à 8, **caractérisés en ce qu'**ils présentent, en moyenne, au moins deux unités répétitives de la formule (I).

**10.** Composés copolymères de polyamino-polysiloxane et/ou de polyammonium-polysiloxanen selon l'une des revendications 1 à 9, **caractérisés en ce que** le groupe $V^1$ est sélectionné parmi des radicaux d'hydrocarbure divalents, linéaires, cycliques ou ramifiés, saturés, non saturés ou aromatiques ayant jusque 600, de préférence jusque 400 atomes de carbone, qui peuvent éventuellement contenir un ou plusieurs groupes sélectionnés parmi -O-, -CONH-, $-CONR^2-$, dans laquelle $R^2$ est tel que défini ci-dessus, -C(O), -C(S)- et $-Z^1-$, où $-Z^1-$ est un groupe de la formule

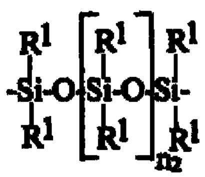

dans laquelle $R^1$ est un $C_1$-$C_{18}$ alkyle, qui peut être éventuellement substitué par un ou plusieurs atomes de fluor, ou un phényle et $n_2$ est tel que défini ci-dessus.

**11.** Composés copolymères de polyamino-polysiloxane et/ou de polyammonium-polysiloxane selon l'une des revendications 1 à 10, **caractérisés en ce que** le groupe $V^1$ est sélectionné parmi :

- $-R^9-$,

dans laquelle $R^9$ est un radical d'hydrocarbure divalent, saturé ou mono- ou polyinsaturé, linéaire ou ramifié ayant de 2 à 25 atomes de carbone,

- **$-(CH_2)\,C\,(O)\,O-[(CH_2CH_2O)_q-(CH_2CH\,(CH_3)\,O)_r]-C\,(O)\,(CH_2)-$**
- **$-(CH_2)_uC\,(O)\,O-R^9-O-C\,(O)\,(CH_2)_u-$,** dans laquelle $R^9$ est tel que défini ci- dessus,
- **$-(CH_2)_u-R^{10}-(CH_2)_u-$,** dans laquelle $R^{10}$ est un groupe aromatique,
- **$-[CH_2CH_2O]_q-[CH_2CH\,(CH_3)\,O]_r-CH_2CH_2-$,**
- **$-CH\,(CH_3)\,CH_2O\,[CH_2CH_2O]_q-[CH_2CH\,(CH_3)\,O]_r-CH_2CH\,(CH_3)-$**
- **$-CH_2\,(OH)\,CH_2$-,**

■ **$- CH_2CH (OH) (CH_2)_2CH (OH) CH_2-$,**
■ **$- CH_2CH (OH) CH_2OCH_2CH (OH) CH_2OCH_2CH (OH) CH_2-$** et
■ **$- CH_2CH (OH) CH_2O- [CH_2CH_2O]_q- [CH_2CH (CH_3) O]_r- CH_2CH (OH) CH_2-$**

dans lesquelles
u est de 1 à 3,
q et r sont de 0 à 200, de préférence de 0 à 100, plus préférablement de 0 à 70 et plus préférablement de 0 à 40 et $q+r > 0$.

**12.** Procédé de production des composés copolymères de polyamino-polysiloxane et/ou de polyammonium-polysiloxane selon l'une des revendications 1 à 11, **caractérisé en ce qu'**on

a) fait réagir une amine primaires contenant un groupe de l'oxyde de polyalkylène aussi qu'éventuellement autres composés d'amine avec des composés contenant de polysiloxane amino-réactif et éventuellement des autres composés amino-réactif ou
b) alkyle le groupe amino d'un composé copolymère de polyamino-polysiloxane avec un agent de l'alkylation, qui contient un groupe de l'oxyde de polyalkylène.

**13.** Utilisation des composés copolymères de polyamino-polysiloxane et/ou de polyammonium-polysiloxane selon l'une des revendications 1 à 11 pour le traitement des fibres ou l'équipement des fibres.

**14.** Utilisation des composés copolymères de polyamino-polysiloxane et/ou de polyammonium-polysiloxane selon l'une des revendications 1 à 11 pour le premier équipement et le traitement des textiles et des autres matériaux naturels ou synthétiques fibreux, incluant du papier, des cheveux et de la laine.

**15.** Utilisation des composés copolymères de polyamino-polysiloxane et/ou de polyammonium-polysiloxane selon l'une des revendications 1 à 11 pour le traitement des textiles et des autres matériaux naturels et synthétiques fibreux dans des agents de prétraitement des fibres et en particulier dans des formulations détergentes contenant des tensioactifs non-ioniques et/ou anioniques.

**16.** Utilisation des composés copolymères de polyamino-polysiloxane et/ou de polyammonium-polysiloxane selon l'une des revendications 1 à 11 en tant qu'un constituant des systèmes d'adoucissants séparés après le lavage des fibres et des textiles, en tant qu'aide au repassage et agent permettant d'empêcher ou d'annuler des bouloches.

**17.** Utilisation des composés copolymères de polyamino-polysiloxane et/ou de polyammonium-polysiloxane selon l'une des revendications 1 à 11 pour le traitement et l'équipement des surfaces dures, telles que le verre, la céramique, le carrelage, des surfaces en plastique, des surfaces métalliques, des surfaces de laque, en particulier des carrosseries de voiture, plus particulièrement dans des formulation de séchage pour les systèmes mécaniques de lavage de voiture.

**18.** Utilisation des composés copolymères de polyamino-polysiloxane et/ou de polyammonium-polysiloxane selon l'une des revendications 1 à 11 dans des systèmes cosmétiques pour le traitement des cheveux et de la peau.

**19.** Émulsion aqueuse contenant au moins un composé copolymère de polyamino-polysiloxane et/ou de polyammonium-polysiloxane selon l'une des revendications 1 à 11 et éventuellement un ou plusieurs agents tensioactifs et éventuellement un ou plusieurs composés de polysiloxanes exempte de l'azote.

**20.** Utilisation de l'émulsion aqueuse selon la revendication 19 dans des applications, qui sont définies dans des revendications 13 à 18.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 441530 A
- US 5807956 A
- US 5981681 A
- US 6475568 B
- US 5591880 A
- US 5650529 A
- DE 3719086 A
- EP 282720 A
- US 6240929 B
- US 5098979 A
- US 5153294 A
- US 5166297 A
- US 5602224 A
- US 6242554 B
- WO 0210257 A
- WO 0210259 A
- US 20020103094 A
- WO 0378504 A
- DE 10214290
- DE OS3340708 A
- DE OS10036533 A
- WO 03078504 A
- WO 2004041912 A
- WO 2004042136 A
- EP 2004050472 W